(19) Europäisches Patentamt
European Patent Office
Office européen des brevets

(11) EP 1 658 309 B1

(12) **FASCICULE DE BREVET EUROPEEN**

(45) Date de publication et mention de la délivrance du brevet:
**20.12.2006 Bulletin 2006/51**

(21) Numéro de dépôt: **04767906.3**

(22) Date de dépôt: **20.07.2004**

(51) Int Cl.:
***C07K 14/435*** *(2006.01)*

(86) Numéro de dépôt international:
**PCT/FR2004/050345**

(87) Numéro de publication internationale:
**WO 2005/010038 (03.02.2005 Gazette 2005/05)**

(54) **PEPTIDE INHIBITEUR DE LA TRADUCTION DES PROTEINES ET SES UTILISATIONS POUR LE CONTROLE DE LA TRADUCTION DES PROTEINES**

PEPTID-INHIBITOR DER PROTEINTRANSLATION UND DESSEN VERWENDUNG BEI DER STEUERUNG DER PROTEINTRANSLATION

PEPTIDE PROTEIN TRANSLATION INHIBITOR AND THE USE THEREOF FOR PROTEIN TRANSLATION CONTROL

(84) Etats contractants désignés:
**AT BE BG CH CY CZ DE DK EE ES FI FR GB GR HU IE IT LI LU MC NL PL PT RO SE SI SK TR**

(30) Priorité: **21.07.2003 FR 0350357**

(43) Date de publication de la demande:
**24.05.2006 Bulletin 2006/21**

(73) Titulaires:
- **CENTRE NATIONAL DE LA RECHERCHE SCIENTIFIQUE (CNRS)
75794 Paris Cedex 16 (FR)**
- **UNIVERSITE DE RENNES I
F-35065 Rennes (FR)**

(72) Inventeurs:
- **COSSON, Bertrand
F-35290 Le Crouais (FR)**
- **PAILLARD, Luc
F-35520 Montreuil Le Gast (FR)**
- **LEGAGNEUX, Vincent
F-35000 Rennes (FR)**
- **OSBORNE, Howard
F-35580 Saint Senoux (FR)**

(74) Mandataire: **Catherine, Alain et al
Cabinet Harlé et Phélip
7, rue de Madrid
F-75008 Paris (FR)**

(56) Documents cités:

- **PAILLARD LUC ET AL: "East of EDEN was a poly (A) tail." BIOLOGY OF THE CELL (PARIS), vol. 95, no. 3-4, mai 2003 (2003-05), - juin 2003 (2003-06) pages 211-219, XP002262151 ISSN: 0248-4900**
- **MINSHALL NICOLA ET AL: "A conserved role of a DEAD box helicase in mRNA masking" RNA (NEW YORK), vol. 7, no. 12, décembre 2001 (2001-12), pages 1728-1742, XP001156011 ISSN: 1355-8382**
- **COLLER JEFFERY ET AL: "Tethered function assays using 3' untranslated regions" METHODS (ORLANDO), vol. 26, no. 2, février 2002 (2002-02), pages 142-150, XP002262153 ISSN: 1046-2023 cité dans la demande**
- **TAKAHASHI NOBUHIRO ET AL: "Coexpression of the CUG-binding protein reduces DM protein kinase expression in COS cells" JOURNAL OF BIOCHEMISTRY (TOKYO), [Online] vol. 130, no. 5, novembre 2001 (2001-11), pages 581-587, XP002262154 ISSN: 0021-924X Extrait de l'Internet: URL:http://jb.bcasj.or.jp/130-5/5faawjtx.h tm> [extrait le 2003-11-20]**
- **GOOD PETER J ET AL: "A family of human RNA-binding proteins related to the Drosophila Bruno translational regulator" JOURNAL OF BIOLOGICAL CHEMISTRY, vol. 275, no. 37, 15 septembre 2000 (2000-09-15), pages 28583-28592, XP002262155 ISSN: 0021-9258**
- **CASTAGNETTI STEFANIA ET AL: "Control of oskar mRNA translation by Bruno in a novel cell-free system from Drosophila ovaries" DEVELOPMENT (CAMBRIDGE), vol. 127, no. 5, mars 2000 (2000-03), pages 1063-1068, XP002262156 ISSN: 0950-1991**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 658 309 B1

## Description

## DOMAINE DE L'INVENTION

La présente invention se rapporte au domaine de la régulation de la synthèse des protéines par contrôle des étapes post-transcriptionnelles de traduction des ARN messagers en protéines. Les principales applications industrielles de l'invention sont le contrôle de la production de protéines d'intérêt en bioréacteur, le contrôle de la production de protéines d'intérêt en thérapie cellulaire (thérapie génique somatique), ou encore le contrôle de la production de protéines virales dans le cadre de thérapies antivirales.

## ART ANTERIEUR

De manière générale, il existe un besoin dans l'état de la technique de disposer de systèmes de contrôle de la production de protéines cibles d'intérêt, en particulier dans le domaine de la production de protéines en bioréacteur ainsi que dans divers types de thérapies médicales.

Dans certains cas, on recherche une inhibition générale de la synthèse des protéines produites par certaines cellules que l'on désire éliminer, comme par exemple des cellules tumorales.

Dans d'autres cas, on cherche à stimuler, ou au contraire à inhiber, la production de seulement une ou plusieurs protéines cibles prédéterminées, comme par exemple des protéines d'intérêt thérapeutique dont on désire l'expression contrôlée à des moments bien déterminés, par exemple dans des procédés de production de protéines d'intérêt par des cellules cultivées dans des bioréacteurs, ou encore par des cellules utilisées en thérapie génique cellulaire somatique.

On connaît, dans l'état de la technique, des protéines capables d'agir, au niveau post-transcriptionnel, sur le niveau de traduction des protéines par les cellules. L'activité biologique post-transcriptionnelle de certaines protéines peut consister en une modification du métabolisme des ARN messagers, par exemple par modification de la stabilité et de la demi-vie des ARN messagers, par une activation de la traduction des ARN messagers, ou encore par une modification du transport ou de la localisation des ARN messagers.

Ainsi, il a été montré que des protéines originaires de levure, telles que Pab1p, Pub1p, She2p, She3p ; originaires du xénope telles que Xp54 et PAP1 ; ou encore des protéines de mammifères telles que hUPF1, hUPF2, hUPF3a, hUPF3b, RNP S1, Y14, DEK, REF2, SRm160, eIF-4E, eIF-4G, REV, TAP1 et NXF3 étaient capables, après liaison spécifique à l'ARN messager, de modifier le métabolisme de l'ARN messager sur lequel ces protéines se fixent, par exemple en stabilisant cet ARN messager, en stimulant la traduction de l'ARN messager, en stimulant l'export noyau-cytoplasme de l'ARN messager ou en stimulant la polyadénylation de l'ARN messager (COLLER et al., 2002).

Pour tester l'activité biologique des différentes protéines susceptibles d'agir sur les diverses étapes post-transcriptionnelles de l'expression des gènes, c'est-à-dire sur les différents aspects du métabolisme des ARN messagers évoqués ci-dessus, il a été proposé, dans l'état de la technique, de fusionner la protéine dont la fonction est testée avec une protéine de liaison à l'ARN de spécificité connue, telle que la protéine MS2CP. L'activité de la protéine de fusion (protéine testée-protéine de liaison à l'ARN) est testée sur une construction d'ADN rapporteur codant un ARN messager comprenant (i) le motif nucléotidique cible de la protéine de liaison à l'ARN et (ii) un cadre ouvert de lecture codant une protéine rapporteur, comme la luciférase ou la bêta-globine (COLLER et al., 2002, Demande PCT N°WO 99/60.408).

Il a également été proposé d'utiliser un polypeptide de fusion comprenant une protéine de fixation à l'ARN fusionnée à une protéine dérivée du facteur eIF4G, afin d'activer spécifiquement la traduction de protéines d'intérêt (Demande PCT N°WO 00/53.779).

La protéine EDEN-BP de Xe*nopus laevis* a été le premier facteur agissant en *trans* pour lequel il a été démontré directement un rôle essentiel dans la spécificité de désadénylation des ARN messagers (PAILLARD et al., 1998). Ultérieurement, il a été montré que la protéine CUG-BP, qui est codée par le gène humain orthologue du gène codant EDEN-BP, est vraisemblablement un facteur à fonction de désadénylation responsable du contrôle post-transcriptionnel de l'ARN messager du proto-oncogène c-Jun dans les cellules de mammifères (PAILLARD et al., 2002).

Il existe un besoin dans l'état de la technique pour des constructions codant une protéine capable d'agir sur l'ARN messager afin d'inhiber la traduction des protéines en général, ou encore pour inhiber la traduction de protéines cibles prédéterminées.

## SOMMAIRE DE L'INVENTION

Il est fourni selon l'invention un peptide inhibiteur de la traduction des protéines caractérisé en ce que sa taille est d'au plus 250 acides aminés et en ce qu'il comprend une séquence d'acides aminés possédant au moins 85 pour cent d'identité avec la séquence en acides aminés SEQ ID N°1.

Un autre objet de l'invention consiste en un polypeptide de fusion susceptible d'inhiber spécifiquement la traduction d'un polynucléotide cible d'intérêt, caractérisé en ce que ledit polypeptide comprend un peptide inhibiteur tel que défini ci-dessus, ledit peptide inhibiteur étant fusionné avec une protéine de liaison à l'ARN.

L'invention fournit également des acides nucléiques comprenant un polynucléotide codant le peptide inhibiteur ou encore le polypeptide de fusion définis ci-dessus.

L'invention est également relative à un système de contrôle de la traduction d'un polynucléotide d'intérêt comprenant :

(a) un premier acide nucléique consistant en un acide nucléique codant un peptide de fusion tel que défini ci-dessus ;
(b) un second acide nucléique comprenant :

(i) au moins une copie d'une séquence nucléotidique cible de la protéine de liaison à l'ARN contenue dans le polypeptide de fusion codé par le premier acide nucléique tel que défini en (a) ;
(ii) le polynucléotide d'intérêt.

Elle a également trait à des vecteurs dans lesquels sont insérés les divers acides nucléiques inclus dans le système de contrôle de la traduction d'un polynucléotide d'intérêt défini ci-dessus, ainsi qu'à des procédés pour contrôler *in vitro* la traduction d'un polynucléotide cible d'intérêt, lesdits procédés mettant en oeuvre le système de contrôle de la traduction définie ci-dessus.

L'invention concerne aussi des kits ou trousses destinés au contrôle de la traduction d'un polynucléotide cible d'intérêt.

L'invention se rapporte également à l'utilisation d'un système de contrôle ou d'un kit tel que défini ci-dessus pour le contrôle de la traduction d'un polynucléotide cible d'intérêt.

L'invention est également relative à des compositions pharmaceutiques comprenant un polypeptide de fusion comprenant un peptide inhibiteur de la traduction des protéines, ledit peptide étant fusionné avec une protéine de liaison à l'ARN, comme décrit ci-dessus.

## DESCRIPTION DES FIGURES

La figure **1** illustre un schéma d'action d'un polypeptide de fusion selon l'invention comprenant le peptide inhibiteur Pep58X fusionné avec la protéine de liaison à l'ARN MS2CP. Le polypeptide de fusion est annoté « MS2CP-Pep58X ». En bas de la figure est représenté un ARN messager cible comprenant le motif nucléotidique cible de la protéine de liaison à l'ARN MS2CP, qui a été désigné « MS2 », et qui est représenté sur la figure par une structure en tige-boucle, et une cassette d'expression de la protéine cible d'intérêt. Comme représenté sur la figure, la protéine MS2CP fusionnée permet la fixation du polypeptide de fusion sur l'ARN messager cible au niveau du site MS2, ce qui permet au peptide inhibiteur Pep58X d'exercer son activité biologique inhibitrice spécifiquement sur l'ARN messager cible.

La figure **2** est un schéma représentant la carte du plasmide pMS2CP-Pep58X. Sur le vecteur, « CMV » désigne le promoteur du virus de la mosaïque du chou-fleur, qui contrôle l'expression du cadre ouvert de lecture codant le polypeptide de fusion entre la protéine de liaison et l'ARN MS2CP et le peptide inhibiteur Pep58X. Sur la figure, le cadre de lecture ouvert code pour un polypeptide de fusion dans lequel la protéine MS2CP et le peptide inhibiteur Pep58X sont séparés par le peptide HA (séquence « YPYDVPDYA » [SEQ ID N°11] allant de l'acide aminé 98 à l'acide aminé 106 de la protéine hémaglutinine HA1), qui constitue une étiquette de détection et de purification du polypeptide de fusion. « T7 » désigne le promoteur du phage T7, qui permet la synthèse d'ARN *in vitro.*

Le vecteur comprend également un gène de résistance à la néomycine (« $Neo^r$ ») placé sous le contrôle du promoteur du virus SV40.

Le vecteur comprend également un cadre ouvert de lecture codant pour une protéine de résistance à l'ampicilline.

L'extrémité 3' du cadre ouvert de lecture codant le polypeptide de fusion comprend une séquence signal de polyadénylation dérivée de l'ADNc codant l'hormone de croissance bovine BGH (pour « Bovine Growth Hormone »).

La figure **3** est un schéma du vecteur rapporteur pRLucLuc-CMVin + 3'UTRGb (MS2)n.

Sur la figure, le promoteur CMV bidirectionnel contrôle l'expression de deux cadres ouverts de lecture, respectivement (i) un cadre ouvert de lecture codant la luciférase R (« Luc R ») et comprenant une région 3'UTR contenant huit copies du site nucléotidique MS2, site de reconnaissance de la protéine MS2CP et (ii) un cadre de lecture ouvert codant la protéine luciférase F(« LucF »).

Le vecteur comprend également un gène de résistance à l'ampicilline (« $Amp^r$ »), placé sous le contrôle du promoteur CMV.

La figure **4** illustre un schéma de fonctionnement d'un système d'expression I.

La figure **4A** illustre la synthèse du polypeptide de fusion Pep58X-protéine de liaison à l'ARN lorsque le promoteur contenant la séquence TetP est activé par la protéine activatrice tTA, qui est elle-même exprimée de manière constitutive.

La figure **4B** illustre l'absence de production du polypeptide de fusion Pep58X-protéine de liaison à l'ARN dans une situation dans laquelle la protéine activatrice tTA est produite en présence de tétracycline et n'active pas la séquence TetP du promoteur contrôlant l'expression du gène Pep58X-protéine de liaison à l'ARN.

La figure **5** illustre le principe de fonctionnement du système d'expression II.

La figure **5A** illustre la répression de l'expression du polypeptide de fusion Pep58X-protéine de liaison à l'ARN sous l'effet de l'inhibition du promoteur contenant la séquence TetO par la protéine répresseur Tet(r), qui est elle-même exprimée de manière constitutive.

La figure **5B** illustre l'activation de la synthèse du polypeptide de fusion Pep58X-protéine de liaison à l'ARN lorsque la protéine répresseur Tet(r) est mise en contact avec la tétracycline, qui le désactive et l'empêche d'inhiber le promoteur contenant la séquence TetO.

La figure **6** illustre les résultats d'activité inhibitrice d'un peptide inhibiteur de l'invention sur la traduction de la protéine rapporteur CAT chez le Xénope.

Fig. **6A** : expression de l'ARNm CAT-82 en l'absence de peptide, ou en présence des peptides Pep58X (« 58 »), Pep60X (« 60 ») et Pep61X (« 61 »).

Fig. **6B** expression des ARNm CAT-B1 (« B1 ») et CAT-B2 (« B2 ») en l'absence de peptide, ou en présence du peptide Pep58X (« B1 + 58 » et « B2+58 », respectivement).

En abscisse : identité du peptide utilisé (figures 6A et 6B) et identité de l'ARNm CAT utilisé (figure 6B).

En ordonnées : Activité CAT, exprimée en pourcentage de l'activité sans peptide, l'activité sans peptide ayant été fixée arbitrairement à 100%.

La figure **7** illustre les résultats de la traduction de la protéine marqueur luciférase en mettant en oeuvre un système de contrôle de la traduction incluant les vecteurs pMS2CP-Pep58X, pMS2CP (utilisé comme contrôle), et le vecteur rapporteur pRLuc Luc CMVin + 3'UTRgb $(MS2)_8$.

En abscisse : concentration de protéines exprimées. En ordonnées : expression de Luc F (fig. 7A) et de Luc R rapportée à l'expression de Luc F (fig. 7B).

## DESCRIPTION DETAILLEE DE L'INVENTION

Il est montré selon l'invention qu'une petite famille de peptides, qui possèdent entre eux une forte homologie structurale, a la capacité d'inhiber la traduction des protéines dans des systèmes cellulaires

Plus spécifiquement, le demandeur a montré que des peptides particuliers, dérivés d'une région spécifique des protéines EDEN-BP et CUG-BP, possédaient une activité d'inhibition de la traduction des protéines, lorsque ces peptides sont co-injectés avec un ARNm rapporteur dans des cellules en culture.

Il a ainsi été montré selon l'invention qu'un peptide de 28 acides aminés possédant la séquence d'acides aminés allant de l'acide aminé en position 183 jusqu'à l'acide aminé en position 210 de la séquence de la protéine EDEN-BP de 489 acides aminés de longueur (SEQ ID n°9), originaire de X*enopus laevis,* possédait, à lui seul, l'activité inhibitrice de la traduction des protéines recherchée.

Ce premier peptide de 28 acides aminés est désigné « Pep58X » aux fins de la présente description.

Il a également été montré selon l'invention qu'un peptide possédant un très haut degré d'identité en acides aminés avec le peptide inhibiteur Pep 58X défini ci-dessus, ledit peptide étant désigné Pep58H, possédait également l'activité inhibitrice de la traduction des protéines qui est recherchée.

Le peptide Pep58H consiste en un peptide de 28 acides aminés de longueur possédant la séquence d'acides aminés allant de l'acide aminé en position 183 jusqu'à l'acide aminé en position 210 de la protéine CUGBP humaine.

On a montré dans les exemples que le niveau d'activité inhibitrice de Pep58X et de Pep58H est très similaire. On a aussi montré que le niveau d'activité inhibitrice de Pep58H est identique à celui observé pour la protéine CUGBP complète.

A l'inverse, il a aussi été montré selon l'invention que d'autres peptides de 28 acides aminés de longueur et dérivés de la région de 84 acides aminés allant de l'acide aminé en position 155 jusqu'à l'acide aminé en position 238 de la protéine EDEN-BP de séquence SEQ ID N°9 ne possédait pas d'activité inhibitrice sur la traduction des protéines. Il s'agit notamment des peptides désignés respectivement Pep60X et Pep61X dans les exemples.

### Famille de peptides inhibiteurs non spécifiques selon l'invention

On a montré selon l'invention que le peptide de 28 acides aminés dérivé de la protéine EDEN-BP de X*énopus laevis,* désigné Pep58X dans la présente description, et qui possède la séquence en acides aminés SEQ ID N°1, est capable d'inhiber, de manière non spécifique, la traduction d'ARN messagers rapporteurs codant pour l'enzyme CAT (chloramphénicol acétyle transférase).

Ainsi, le peptide Pep58X a été co-injecté avec différents ARN messagers rapporteurs dans des embryons de l'amphibien *Xénope* (Xe*nopus laevis).* Après expression des gènes rapporteurs, des extraits protéiques ont été préparés et l'activité du gène rapporteur a été dosée. Le peptide Pep58X inhibe la traduction des ARN messagers rapporteurs codant pour l'enzyme CAT, que la construction d'ARN messager rapporteur comprenne également le site nucléotidique EDEN de liaison à la protéine EDEN-BP ou que l'ARN messager rapporteur ne comprenne aucun site nucléotidique EDEN spécifique de la liaison de l'ARN à la protéine EDEN-BP. En conséquence, il est montré selon l'invention qu'un peptide comprenant la séquence en acides aminés SEQ ID N°1, comme c'est le cas du peptide Pep58X, inhibe de manière générale et non spécifique la traduction des ARN messagers cellulaires.

L'activité inhibitrice générale non spécifique de la traduction des protéines démontrée avec les peptides Pep58X de séquence SEQ ID N°1 et Pep58H de séquence SEQ ID N°2, qui possèdent entre eux une forte identité de séquence en acides aminés d'environ 89,3%, a permis au demandeur de définir une famille restreinte de peptides inhibiteurs de la traduction des protéines, qui est décrite ci-dessous.

La taille des peptides inhibiteurs non spécifiques de la traduction des ARN messagers en protéines selon l'invention, qui possèdent tous une forte identité de séquence en acides aminés avec le peptide Pep58X, est d'au plus 250 acides aminés.

Bien que des peptides d'une longueur supérieure à 250 acides aminés pourraient aussi posséder des propriétés inhibitrices de la traduction des protéines, le demandeur pense, sans vouloir être lié par une quelconque théorie, que de tels peptides de grande taille sont susceptibles de posséder une activité inhibitrice réduite, notamment du fait de la création de contraintes de conformation du peptide.

L'invention a donc pour objet un peptide inhibiteur de la traduction des protéines, caractérisé en ce que sa taille d'au plus 250 acides aminés et en ce qu'il comprend une séquence d'acides aminés possédant au moins 85% d'identité avec la séquence en acides aminés SEQ ID N°1 de Pep58X.

Les termes protéine, polypeptide et peptide utilisés dans la présente description sont interchangeables et désignent une chaîne linéaire de résidus d'acides aminés liés l'un à l'autre par une liaison peptidique entre le groupe alpha-aminé et le groupe carboxy de deux résidus d'acides aminés contigus. Font également partie de l'invention des polypeptides comprenant au moins une liaison non peptidique telle qu'une liaison rétro-inverso (NHCO), une liaison carba($CH_2$-$CH_2$) ou encore une liaison cétométhylène (CO-$CH_2$).

Un peptide inhibiteur de la traduction des protéines selon l'invention comprend une séquence d'acides aminés possédant de préférence au moins 86%, 87%, 88%, 89%, 89,3%, 90%, 91%, 92%, 93%, 94%, 95%, 96%, 97%, 98%, ou 99% d'identité en acides aminés avec le peptide inhibiteur de séquence SEQ ID N°1.

Le « pourcentage d'identité » entre deux séquences d'acides aminés, au sens de la présente invention, est déterminé en comparant les deux séquences alignées de manière optimale, à travers une fenêtre de comparaison.

La partie de la séquence d'acides aminés dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal entre les deux séquences.

Le pourcentage d'identité entre les deux séquences d'acides aminés est calculé en déterminant le nombre de positions auquel un résidu d'acide aminé identique est observé pour les deux séquences comparées après alignement, puis en divisant le nombre de positions auxquelles il y a identité entre les deux résidus d'acides aminés comparés, par le nombre total de positions dans la fenêtre de comparaison, puis en multipliant le résultat par cent afin d'obtenir le pourcentage d'identité entre les deux séquences d'acides aminés.

L'alignement optimal des séquences pour la comparaison peut être réalisé de manière informatique à l'aide d'algorithmes connus.

De manière tout à fait préférée, le pourcentage d'identité de séquence est déterminé à l'aide du logiciel CLUSTAL W (version 1.82) les paramètres étant fixés comme suit : (1) CPU MODE = « ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » (6) KTUP (WORD SIZE) = « default » ; (7) WINDOW LENGTH = « default » ; (8) SCORE TYPE = « percent » ; (9) TOPDIAG= « default » ; (10) PAIRGAP= « default » ; (11) PHYLOGENETIC TREE/TREE TYPE = « none » ; (12) MATRIX = « default » ; (13) GAP OPEN = « default » ; (14) END GAPS = «default»; (15) GAP EXTENSION = «default»; (16) GAP DISTANCES = « default » (17) TREE TYPE = « cladogram » et (18) TREE GRAP DISTANCES = « hide ».

Les peptides appartenant à la famille des peptides inhibiteurs de la traduction des protéines selon l'invention possèdent donc, pour certains d'entre eux, une séquence d'acides aminés comprenant une ou plusieurs substitutions, additions, ou délétions d'un acide aminé, par rapport aux peptides inhibiteurs comprenant la séquence SEQ ID N°1. Un exemple illustratif de tels peptides est le peptide inhibiteur de séquence SEQ ID N°2.

Font partie de l'invention les peptides dont la séquence d'acides aminés possède une ou plusieurs substitutions d'un acide aminé par un acide équivalent, par rapport aux peptides inhibiteurs de la traduction des protéines telles que définis de manière générale ci-dessus.

On entendra par acides aminés équivalents selon la présente invention des acides aminés qui ont une activité inhibitrice du même ordre de grandeur que l'activité inhibitrice du peptide inhibiteur de référence, c'est-à-dire des acides aminés, lesquels, lorsqu'ils remplacent un acide aminé présent dans la séquence d'acides aminés du peptide inhibiteur de référence, ont une activité inhibitrice ayant le même ordre de grandeur que celui du peptide inhibiteur de référence.

A titre d'illustration, des substitutions conservatives d'acides aminés sont les remplacements d'un acide aminé par un autre acide aminé appartenant à la même classe. Sont ainsi interchangeables les acides aminés aliphatiques Ala, Val, Leu et Ile ; les acides aminés possédant un résidu hydroxyle tel que Ser et Thr ; les acides aminés acides Asp et Glu ; les acides aminés possédant une fonction amide telle que Asn et Gln ; les acides aminés basiques tels que Lys et Arg ; et les acides aminés aromatiques tels que Phe et Tyr.

Le niveau d'activité inhibitrice d'un peptide inhibiteur selon l'invention peut être aisément déterminé par l'homme du métier, par exemple en calculant, pour chaque concentration d'une série de concentrations croissantes intracellulaires de peptide inhibiteur, le pourcentage d'expression d'une protéine marqueur codée par un ARNm rapporteur, par rapport au niveau d'expression de ladite protéine marqueur en l'absence du peptide inhibiteur, puis en calculant la pente de la droite reliant les différentes valeurs de pourcentage d'expression de la protéine marqueur, pour les différentes valeurs croissantes de concentration du peptide inhibiteur, comme cela est détaillé dans les

exemples.

Le calcul de la pente de la droite d'inhibition peut être réalisé à l'aide de la formule suivante :

$$P = \frac{d[\text{Protéine marqueur}]}{d[\text{peptide inhibiteur}]},$$

dans laquelle

- P est la valeur de la droite de pente d'inhibition ;
- d [protéine marqueur] est la différence de quantité de protéine marqueur entre les deux concentrations en peptides a et b ;
- d [peptide inhibiteur] est la différence entre les deux concentrations a et b de peptide.

Selon l'invention, un peptide inhibiteur déterminé possède un niveau d'activité inhibitrice de la traduction des protéines du « même ordre de grandeur » que le niveau d'activité inhibiteur d'un peptide de référence lorsque la valeur P dudit peptide déterminé est comprise entre- 0,3 et -0,8, de préférence entre - 0,4 et - 0,7, la valeur P étant de préférence d'environ - 0,6.

Font aussi partie de l'invention des peptides d'au plus 250 acides aminés de longueur et qui possèdent au moins 85% d'identité en acides aminés avec la séquence d'acides aminés allant de l'acide aminé en position 156 jusqu'à l'acide aminé en position 405 de la séquence de la protéine EDEN-BP, la protéine EDEN-P possède la séquence en acides aminés référencée dans la présente description comme la séquence SEQ ID n°9. La séquence d'acides aminés 156-405 comprise dans la séquence SEQ ID N°9 de EDEN-BP consiste en la séquence d'acides aminés qui est comprise entre le deuxième et le troisième domaine de liaison à l'ARN de la protéine EDEN-BP.

Font aussi partie de l'invention des peptides d'au plus 100 acides aminés de longueur et qui comprennent au moins 30 acides aminés consécutifs de la séquence de 84 acides aminés allant de l'acide aminé en position 155 jusqu'à l'acide aminé en position 238 de la séquence de la protéine EDEN-BP, référencée comme la séquence SEQ ID N°3, lesdits peptides comprenant une séquence d'acides aminés possédant au moins 85% d'identité en acides aminés avec la séquence SEQ ID N°1.

Font également partie de l'invention des peptides d'au plus 100 acides aminés de longueur et qui comprennent au moins 30 acides aminés consécutifs la séquence d'acides aminés de 88 acides aminés allant de l'acide aminé en position 155 à l'acide aminé en position 242 de la séquence de la protéine CUG-BP, référencée comme la séquence SEQ ID N°4 selon l'invention, lesdits peptides comprenant une séquence d'acides aminés possédant au moins 85% d'identité en acides aminés avec la séquence SEQ ID N°2.

Le demandeur a en effet montré que la séquence SEQ ID N°3 de 84 acides aminés, dérivée de la protéine EDEN-BP, interagit dans la cellule avec la protéine ePABP, connue pour être impliquée dans les mécanismes de régulation du niveau de la traduction des ARN messagers.

Un peptide inhibiteur de la traduction des protéines tel que défini ci-dessus a avantageusement une taille d'au plus 50 acides aminés de longueur.

Préférentiellement, un peptide inhibiteur de la traduction des protéines de l'invention est caractérisé en ce que sa taille est d'au plus 30 acides aminés de longueur et est de manière tout à fait préférée d'au plus 28 acides aminés de longueur.

Les peptides inhibiteurs de petite taille, d'au plus 50 acides aminés et mieux d'au plus 30 acides aminés, sont préférés du fait de leur meilleure capacité à pénétrer les membranes cellulaires et agir ainsi efficacement et rapidement au niveau du cytoplasme pour inhiber la traduction des ARN messagers.

Fait partie de l'invention un peptide inhibiteur de la traduction des protéines qui est caractérisé en ce qu'il comprend une séquence d'acides aminés possédant au moins 89%, de préférence 89,3% , d'identité avec la séquence en acides aminés SEQ ID N°1.

Répond à la définition d'un peptide inhibiteur ci-dessus un peptide caractérisé en ce qu'il comprend la séquence SEQ ID N°1, en particulier le peptide de séquence SEQ ID N°1.

Fait également partie de la définition un peptide ci-dessus, un peptide caractérisé en ce qu'il comprend la séquence SEQ ID N°2, en particulier le peptide de séquence SEQ ID N°2.

L'invention est également relative à un procédé pour la production d'un peptide inhibiteur de la traduction des protéines tel que défini ci-dessus, ledit procédé comprenant les étapes de :

(a) insérer un acide nucléique codant pour ledit peptide inhibiteur dans un vecteur d'expression approprié ;
(b) cultiver, dans un milieu de culture approprié, une cellule hôte préalablement transformée ou transfectée avec le vecteur recombinant obtenu à l'étape (a) ;
(c) récupérer le milieu de culture conditionné ou lyser la cellule hôte, par exemple par sonication ou par choc osmotique ;
(d) séparer et purifier, à partir dudit milieu de culture, ou encore à partir des lysats cellulaires obtenus à l'étape (c), ledit peptide inhibiteur ;
(e) le cas échéant, caractériser le peptide inhibiteur recombinant produit.

Les peptides inhibiteurs selon l'invention peuvent être caractérisés par fixation sur une colonne de chromatographie d'immunoaffinité sur laquelle des anticorps dirigés contre ledit peptide ont été préalablement immobilisés.

Selon un autre aspect, un peptide inhibiteur selon l'invention peut être purifié par passage sur une série appropriée de colonnes de chromatographie, selon des méthodes connues de l'homme du métier et décrites par exemple dans AUSUBEL et al. (1989). Un peptide inhibiteur selon l'invention peut également être préparé par les techniques classiques de synthèse chimique, indifféremment en solution homogène ou en phase solide.

A titre illustratif, un peptide inhibiteur selon l'invention pourra être préparé par la technique en solution homogène décrite par HOUBEN WEIL (1974) ou encore par la technique de synthèse en phase solide décrite par MERRIFIELD (1965a, 1965b).

L'invention est également relative à un acide nucléique comprenant un polynucléotide codant un peptide inhibiteur de la traduction des protéines tel que défini ci-dessus.

De préférence, ledit acide nucléique est caractérisé en ce qu'il comprend un polynucléotide régulateur sous le contrôle duquel est placé le polynucléotide codant ledit peptide inhibiteur de la traduction des protéines.

L'invention concerne aussi un acide nucléique tel que défini ci-dessus, caractérisé en ce qu'il est inséré dans un vecteur recombinant de clonage ou d'expression.

L'invention est également relative à un vecteur de clonage ou d'expression recombinant comprenant un acide nucléique tel que défini ci-dessus.

Tout vecteur approprié connu ou défini dans la présente description peut être utilisé.

Les peptides inhibiteurs tels que définis ci-dessus sont utilisés pour inhiber de manière non spécifique la traduction des protéines, de préférence dans des systèmes cellulaires, bien que les peptides inhibiteurs puissent être également utilisés pour inhiber la traduction des protéines dans des systèmes acellulaires, par exemple à partir d'un lysat de cellules, par exemple en incubant un extrait d'un ARN messagers cellulaires dans un système acellulaire constitué d'un lysat de réticulocytes de lapin selon la technique conventionnelle qui est décrite par UCHIDA et al. (2002). Selon un aspect préféré de l'invention, on utilise les peptides inhibiteurs tels que définis ci-dessus afin de perturber le métabolisme cellulaire au point de provoquer la mort des cellules dans lesquelles ces peptides inhibiteurs ont pénétré.

Ainsi, les peptides inhibiteurs de la traduction des protéines tels que définis ci-dessus peuvent être utilisés pour éliminer certaines catégories de cellules à l'encontre desquelles ils peuvent être ciblés, telles que les cellules tumorales.

Par exemple, les peptides inhibiteurs selon l'invention peuvent être fusionnés avec un peptide ou une protéine ou encore couplés à un polysaccharide, ledit peptide, ladite protéine ou ledit polysaccharide consistant en des ligands reconnus spécifiquement par des récepteurs exprimés dans les cellules cibles, par exemple sur la surface membranaire des cellules cibles, lesdites cellules cibles internalisant alors les peptides inhibiteurs qui vont provoquer leur mort par blocage de la traduction des protéines cellulaires. Des exemples de peptides ou de protéines qui peuvent être fusionnés avec un peptide inhibiteur selon l'invention sont notamment les anticorps ou les fragments d'anticorps qui reconnaissent spécifiquement des antigènes exprimés de manière spécifique par certaines catégories cellulaires, telles que les cellules tumorales.

Par exemple, on peut préparer une protéine de fusion entre un peptide inhibiteur selon l'invention et un anticorps ou un fragment d'anticorps, par exemple un fragment Fab ou encore un fragment $F(ab')_2$ reconnaissant spécifiquement un antigène exprimé sélectivement par les cellules tumorales visées, tel que par exemple l'antigène tumoral Tn bien connu dans l'état de la technique. *In vitro,* les cellules tumorales présentes dans une population de cellules prélevées chez un patient peuvent ainsi être éliminées par incubation des cellules provenant du patient avec des concentrations appropriées de la protéine de fusion peptide inhibiteur-anticorps (ou fragment d'anticorps), suivie d'une récupération des cellules normales non tumorales.

Il va sans dire que les protéines de fusion peptide inhibiteur-ligand d'un récepteur cellulaire peuvent également être utilisées *in vivo,* par exemple dans le cadre de thérapies anticancéreuses.

**Polypeptides de fusion inhibiteurs spécifiques de la traduction des protéines.**

Selon un autre aspect, l'invention est relative à des polypeptides de fusion susceptibles d'inhiber spécifiquement la traduction d'un polynucléotide cible d'intérêt en la protéine correspondante, un tel polypeptide de fusion comprenant un peptide inhibiteur de la traduction des protéines tel que défini précédemment dans la description, ledit peptide inhibiteur étant fusionné avec une protéine de liaison à l'ARN reconnaissant spécifiquement un site nucléotidique cible de l'ARN messager qui est visé.

En effet, il a été montré selon l'invention qu'un polypeptide de fusion entre le peptide Pep58X de séquence SEQ ID N°1 et la protéine de liaison à l'ARN MS2CP était capable d'inhiber spécifiquement la traduction d'un ARN messager comprenant le site nucléotidique cible MS2 et un cadre ouvert de lecture codant la protéine marqueur luciférase, placé sous le contrôle d'un promoteur approprié.

Comme illustré sur la figure 1, la protéine MS2CP contenue dans le polypeptide de fusion se fixe sélectivement sur son site nucléotidique cible MS2, ce qui permet au peptide inhibiteur Pep58X d'inhiber spécifiquement l'expression de l'ARN messager ou des ARN messagers contenant le site nucléotidique cible MS2.

Les mêmes résultats ont été rapportés par le demandeur avec un polypeptide de fusion contenant le peptide inhibiteur Pep58H et la protéine MS2CP de liaison à l'ARN.

Dans un polypeptide de fusion selon l'invention, la protéine de liaison à l'ARN, qui est fusionnée au peptide inhibiteur de la traduction des protéines défini ci-dessus, est choisie, de préférence parmi MS2CP, N, IRP et U1A, listées dans le tableau 1 ci-dessous.

**Tableau 1 : Protéines amarres préférées**

| Amarre | Référence |
|---|---|
| MS2 | J.Coller, N.Gray, M.Wickens, Genes Dev.12 (1998) 3226-3235. Witherell, G., J. Gott, and O. Uhlenbeck. 1991. Specific interaction between RNA phage coat proteins and RNA. *Prog. Nucleic Acids Res. Mol. Biol.* **40**: 185-220 |
| N | E.De Gregorio, T.Preiss, M.W. Hentze, EMBO J.18 (1999) 4865-4874. Tan, R. & Frankel, A. D. (1994) *Biochemistry* **33**, 14579-14585 |
| IRP | E.De Gregorio, J.Baron, T.Preiss, M.Hentze, RNA 7 (2001)106-113. Hentze MW; Kuhne LC. Proc Natl Acad Sci U S A. 1996 Aug 6;93(16):8175-82. Review. PMID:8710843 |
| U1A | A.S. Brodsky, P.A. Silver, RNA 6 (2000) 1737-1749. |

Avantageusement, la protéine de liaison à l'ARN choisie est localisée à l'extrémité $NH_2$-terminale du polypeptide de fusion, bien qu'elle puisse également être localisée à l'extrémité COOH-terminale dudit polypeptide de fusion.

Dans le polypeptide de fusion, la protéine de liaison à l'ARN peut être fusionnée directement au peptide inhibiteur, c'est-à-dire, selon les cas :

- (i) lorsque la protéine de liaison à l'ARN est localisée à l'extrémité $NH_2$-terminale du polypeptide de fusion, le dernier acide aminé en position COOH-terminale de la protéine de liaison à l'ARN est lié chimiquement, de préférence par une liaison peptidique normale, à l'acide aminé localisé en position $NH_2$-terminale du peptide inhibiteur ; ou
- (ii) lorsque la protéine de liaison à l'ARN est localisée en position COOH-terminale du polypeptide de fusion, l'acide aminé localisé en position COOH-terminale du peptide inhibiteur est lié directement, de préférence par une liaison peptidique normale, à l'acide aminé localisé en position $NH_2$ -terminale de la protéine de liaison à l'ARN.

Selon un autre mode de réalisation d'un polypeptide de fusion selon l'invention, la protéine de liaison à l'ARN et le peptide inhibiteur de la traduction des protéines ne sont pas liés directement l'un à l'autre, mais sont au contraire séparés l'un de l'autre, dans le polypeptide de fusion, par une séquence d'acides aminés espaceur, de préférence hydrophobe. La séquence d'acides aminés espaceur possède une taille suffisante pour constituer une région de flexibilité de la molécule protéique permettant une mobilité relative de la protéine de liaison à l'ARN vis-à-vis du peptide inhibiteur.

La taille du peptide espaceur est d'au moins 3 acides aminés de longueur et d'au plus 50 acides aminés de longueur. De préférence la taille du peptide espaceur est comprise entre 5 et 30 acides aminés de longueur, et de manière tout à fait préférée entre 5 et 20 acides aminés de longueur.

De préférence, lorsque la séquence espaceur, ou peptide espaceur, est hydrophobe, ladite séquence espaceur facilite la pénétration du polypeptide de fusion au travers des membranes cellulaires. Dans ce cas, ledit peptide espaceur contient majoritairement des acides aminés hydrophobes tels que les acides aminés valine, leucine ou encore isoleucine.

Dans ce mode de réalisation, le peptide espaceur comprend dans sa séquence préférentiellement au moins 50% d'acides aminés hydrophobes de préférence au moins 60% et de manière tout à fait préférée au moins 80% d'acides aminés hydrophobes.

Selon un mode de réalisation particulier, le peptide espaceur consiste en une chaîne d'acides aminés poly (Alanine), comprenant de 3 à 50, mieux de 5 à 30, avantageusement de 5 à 20, et de manière tout à fait préférée de 5 à 10 résidus Alanine.

Selon un autre aspect, la séquence d'acides aminés espaceur ou peptide espaceur, constitue une étiquette permettant la détection ou encore la purification du polypeptide de fusion présent dans un échantillon. Par exemple, le peptide espaceur peut être constitué du peptide « HA TAG », de séquence SEQ ID N°11, comme décrit dans les exemples.

Des exemples spécifiques et illustratifs de polypeptides de fusion inhibiteurs spécifiques selon l'invention sont constitués par :

- le polypeptide de fusion MS2CP-HA TAG-Pep58X de séquence en acides aminés SEQ ID N°5, qui est codé par l'acide nucléique de séquence SEQ ID N°7.
- le polypeptide de fusion MS2CP-HA TAG-Pep58H de séquence en acides aminés SEQ ID N°6, qui est codé par l'acide nucléique de séquence SEQ ID N°8.

L'invention est également relative à un acide nucléique comprenant un polynucléotide codant un polypeptide de fusion tel que défini ci-dessus.

Les acides nucléiques préférés selon l'invention sont les suivants :

- l'acide nucléique de séquence SEQ ID N°7 codant le polypeptide de fusion MS2-HA TAG-Pep58X;
- l'acide nucléique de séquence SEQ ID N°8 codant le polypeptide de fusion MS2CP-HA TAG-Pep58H.

Selon un aspect préféré, l'acide nucléique codant un polypeptide de fusion selon l'invention est caractérisé en ce qu'il comprend un polynucléotide régulateur sous le contrôle duquel est placé le polynucléotide codant le polypeptide de fusion.

De manière tout à fait préférée, ledit acide nucléique est caractérisé en ce que le polynucléotide régulateur est un polynucléotide régulateur inductible.

En effet, dans la pratique, le contrôle de la traduction de certaines protéines cibles prédéterminées par un polypeptide de fusion inhibiteur spécifique selon l'invention impose qu'à des moments déterminés d'une culture cellulaire, par exemple en bioréacteur, la ou les protéines cibles ne soient pas produites, alors qu'à d'autres moments au contraire la production de la ou des protéines cibles est recherchée.

L'invention concerne aussi un acide nucléique codant un polypeptide de fusion inhibiteur spécifique tel que défini ci-dessus, caractérisé en ce qu'il est inséré dans un vecteur de clonage ou d'expression.

L'invention est également relative à un vecteur de clonage ou d'expression recombinant, caractérisé en ce qu'il comprend un acide nucléique codant un peptide inhibiteur spécifique tel que défini ci-dessus.

Tout vecteur de clonage ou d'expression connu ou décrit dans la présente invention peut être utilisé.

Les polypeptides de fusion inhibiteurs spécifiques ainsi que les acides nucléiques codant ces polypeptides de fusion de l'invention ont permis aux demandeurs de réaliser des systèmes de contrôle de la traduction d'un ou plusieurs polynucléotides cibles d'intérêt, les caractéristiques techniques de ces systèmes de contrôle étant définies ci-dessous.

**<u>Systèmes de contrôle de la traduction d'un ou plusieurs nucléotides cibles d'intérêt.</u>**

Selon un autre aspect, l'invention est relative à des fusions entre peptide et oligonucléotide susceptible d'inhiber spécifiquement la traduction d'un polynucléotide cible d'intérêt en la protéine correspondante. Une telle molécule de fusion comprenant un peptide inhibiteur de la traduction des protéines tel que défini précédemment dans la description, ledit peptide inhibiteur étant fusionné avec un oligonucléotide reconnaissant spécifiquement un site nucléotidique cible d'un ARNm qui est visé. Un exemple de ce type d'oligonucléotide est l'Aptastruc décrit dans la demande PCT n°PCT/FR 95/01036.

Les polypeptides de fusion inhibiteurs spécifiques de la traduction des protéines, tels que définis ci-dessus, peuvent être intégrés dans différents systèmes de contrôle de la traduction d'un ou plusieurs polynucléotides cibles d'intérêt.

Un premier système de contrôle de la traduction d'un ou plusieurs polynucléotides cibles d'intérêt comprend un acide nucléique codant un polypeptide de fusion peptide inhibiteur-protéine de liaison à l'ARN tel que défini ci-dessus.

Un second système de contrôle de l'invention, consiste en un système de contrôle qui comprend un polypeptide de fusion peptide inhibiteur-protéine de liaison à l'ARN tel que défini ci-dessus.

Lorsque l'acide nucléique codant le polypeptide de fusion, éventuellement sous la forme d'un insert d'ADN dans un vecteur recombinant, est utilisé pour transfecter les cellules dans lesquelles le contrôle de la traduction est recherché, le polypeptide de fusion est exprimé, éventuellement de manière inductible. Lorsque le polypeptide de fusion est exprimé dans les cellules ainsi transfectées, ledit polypeptide de fusion se fixe spécifiquement aux ARN messagers cibles qui comprennent le site nucléotidique cible de la protéine de liaison à l'ARN contenue dans ledit polypeptide de fusion, grâce à quoi ledit peptide de fusion inhibe la traduction des protéines codées par lesdits ARN messagers cibles.

De même, lorsque le polypeptide de fusion inhibiteur spécifique est mis en contact avec les cellules cibles, ledit polypeptide de fusion est intemalisé dans le cytoplasme de ces cellules et inhibe la traduction des protéines codées par les ARN messagers cibles contenant, dans leur séquence, le site nucléotidique cible de la protéine de liaison à l'ARN contenue dans ledit polypeptide de fusion.

Selon un troisième système de contrôle de la traduction d'un ou plusieurs polynucléotides cibles d'intérêt selon l'invention, ledit système de contrôle comprend, d'une part, un acide nucléique codant le polypeptide de fusion inhibiteur spécifique et, d'autre part, un second acide nucléique qui constitue l'acide nucléique cible du polypeptide de fusion et qui code pour la protéine d'intérêt dont le contrôle de la traduction est recherché.

Ainsi, l'invention a également pour objet un système de contrôle de la traduction d'un polynucléotide cible d'intérêt comprenant :

(a) un premier acide nucléique consistant en un acide nucléique comprenant un polynucléotide codant un polypeptide de fusion inhibiteur spécifique tel que défini dans la présente description ;

(b) un second acide nucléique comprenant :

(i) au moins une copie d'une séquence nucléotidique cible de la protéine de liaison à l'ARN contenu dans le polypeptide de fusion codé par le premier acide nucléique tel que défini en (a) ; et

(ii) le polynucléotide d'intérêt dont le contrôle de la traduction est recherché.

De manière tout à fait préférée, le troisième système de contrôle selon l'invention ci-dessus est caractérisé en ce que le second acide nucléique, qui code la protéine d'intérêt dont le contrôle de la traduction est recherché, comprend un polynucléotide régulateur sous le contrôle duquel est placé le polynucléotide d'intérêt codant ladite protéine d'intérêt.

Un quatrième système de contrôle de la traduction selon l'invention, consiste en un système de contrôle de la traduction d'un polynucléotide cible d'intérêt qui comprend :

(a) un polypeptide de fusion peptide inhibiteur-protéine de liaison à l'ARN tel que défini précédemment dans la description ;

(b) un acide nucléique comprenant :

(i) au moins une copie d'une séquence nucléotidique cible de la protéine de liaison à l'ARN contenue dans le polypeptide de fusion tel que défini en (a) ; et

(ii) le polynucléotide d'intérêt dont le contrôle de la traduction est recherché.

De préference, l'acide nucléique (b) des systèmes de contrôle de la traduction des protéines définis ci-dessus comprend au moins 2 copies, avantageusement au moins 3 ou 4 copies de la séquence nucléotidique cible de la protéine de liaison à l'ARN contenue dans le polypeptide de fusion de l'invention. Avantageusement, l'acide nucléique (b) comprend 5, 6, 7, 8, 9, 10, 11, 12, 13, 14, 15, 16, 17, 18, 19, 20, 21, 22, 23 ou 24 copies de ladite séquence nucléotide cible. Dans certains cas, l'acide nucléique (b) contient donc jusqu'à 24 copies de ladite séquence nucléotide cible.

L'invention est également relative à un système de contrôle tel que défini ci-dessus, caractérisé en ce que le ou les acides nucléiques qui sont inclus dans celui-ci sont insérés dans un vecteur d'expression recombinant.

L'invention concerne aussi un système de contrôle tel que défini ci-dessus, caractérisé en ce que l'acide nucléique (b) est inséré dans le génome d'une cellule hôte procaryote ou eucaryote.

Le troisième et le quatrième systèmes de contrôle de la traduction selon l'invention permettent de réguler de manière contrôlée l'expression d'un gène préalablement inséré de manière artificielle dans une cellule, ledit gène étant de préférence un gène codant une protéine dont la synthèse doit être contrôlée .

Par exemple, dans le domaine de la production de protéines d'intérêt dans des bioréacteurs, il existe un problème récurrent lié à la toxicité de la protéine d'intérêt que l'on cherche à exprimer vis-à-vis des cellules en phase de croissance. Dans cette application, le système de contrôle de la traduction des protéines tel que défini ci-dessus permet, du fait de la présence du polypeptide de fusion, la répression de l'expression d'un polynucléotide cible d'intérêt pendant la phase de croissance des cellules, puis, si désiré, l'expression de la protéine d'intérêt, une fois le plateau de croissance cellulaire atteint au sein du bioréacteur.

Par rapport aux autres systèmes de contrôle existants, qui agissent au niveau transcriptionnel de la synthèse d'ARN, et dont il est observé en conséquence une grande inertie de réponse, comme par exemple le système TeT on/Tet off, le système de contrôle selon l'invention permet une réponse rapide de répression ou au contraire d'activation de la synthèse des protéines cibles.

Comme on le comprend, toute la potentialité du système de contrôle de la traduction des protéines selon l'invention est atteinte lorsque l'expression du polypeptide de fusion peptide inhibiteur-protéine de liaison à l'ARN est inductible : ainsi, une répression de la synthèse dudit polypeptide de fusion permettra la traduction du polynucléotide cible d'intérêt en la protéine correspondante, alors qu'une activation de l'expression du polypeptide de fusion inhibera la traduction du polynucléotide cible d'intérêt en la protéine correspondante.

Ainsi, pour poursuivre l'objectif de l'invention, il est donc particulièrement avantageux d'utiliser des constructions d'acides nucléiques dans lesquelles le polynucléotide codant pour le polypeptide de fusion de l'invention est placé sous le contrôle d'un polynucléotide régulateur inductible, dont l'activité peut être contrôlée dans le temps.

Selon un autre aspect de l'invention, on utilise les polypeptides de fusion dans le cadre d'un traitement antiviral ciblé, en utilisant, comme protéine de liaison à l'ARN, une protéine qui se' fixe spécifiquement sur des séquences nucléotidiques d'ARNm viraux.

***<u>Système d'expression inductible du polypeptide de fusion, constitutif des systèmes de contrôle de la traduction des protéines selon l'invention.</u>***

L'invention est donc également relative à un acide nucléique comprenant un polynucléotide codant pour un polypeptide de fusion inhibiteur-protéine de liaison à l'ARN tel que défini ci-dessus et qui comprend aussi un polynucléotide régulateur sensible à l'action, directe ou indirecte, d'un signal inducteur, aussi désigné polynucléotide régulateur inductible.

Des systèmes de régulation inductibles utilisables selon l'invention sont représentés respectivement dans les figures 4 et 5.

D'autres systèmes de régulation inductibles, basés sur des principes de régulation identiques à ceux représentés dans les figures 4 et 5 peuvent également être utilisés.

On préfère les systèmes de régulation inductibles listés dans le tableau 2.

De manière tout à fait préférée, un acide nucléique codant un polypeptide de fusion selon l'invention ainsi qu'un acide nucléique comprenant (i) au moins une copie d'une séquence nucléotidique cible de la protéine de liaison à l'ARN contenue dans ledit polypeptide de fusion et (ii) le polynucléotide d'intérêt dont le contrôle de la traduction est recherché, sont insérés dans un vecteur recombinant de clonage ou d'expression.

**<u>VECTEURS SELON L'INVENTION</u>**

Par « vecteur » au sens de la présente invention, on entend une molécule d'ADN ou d'ARN circulaire ou linéaire, qui est indifféremment sous la forme simple brin ou double brin.

Un vecteur recombinant selon l'invention est de préférence un vecteur d'expression.

Il peut s'agir notamment d'un vecteur d'origine bactérienne ou virale.

Dans tous les cas, l'acide nucléique codant le polypeptide de fusion inhibiteur-protéine de liaison à l'ARN selon l'invention est placé sous le contrôle d'une ou plusieurs séquences contenant des signaux de régulation de son expression dans les cellules considérées, soit que les signaux de régulation soient tous contenus dans l'acide nucléique codant ledit polypeptide de fusion, soit que l'un, plusieurs d'entre eux, ou encore tous les signaux de régulation soient contenus dans le vecteur receveur dans lequel l'acide nucléique codant le polypeptide de fusion a été inséré.

Un vecteur recombinant selon l'invention comprend avantageusement des séquences d'initiation et d'arrêt de la transcription appropriées.

En outre, les vecteurs recombinants selon l'invention peuvent inclure une ou plusieurs origines de réplication fonctionnelles dans les cellules hôtes dans lesquelles leur expression est recherchée, ainsi que, le cas échéant, des séquences nucléotidiques marqueurs de sélection.

Les vecteurs recombinants selon l'invention peuvent aussi inclure un ou plusieurs des signaux de régulation de l'expression tels que définis ci-dessus dans la description, y compris des polynucléotides régulateurs inductibles.

Les vecteurs bactériens préférés selon l'invention sont par exemple les vecteurs pBR322(ATCC N°37017) ou encore les vecteurs tels que pAA223-3 (Pharmacia, Uppsala, Suède) et pGEM1(Promega Biotech, Madison, WI, Etats-Unis). On peut encore citer d'autres vecteurs commercialisés tels que les vecteurs pQE70, qQE60, Pqe9 (QUIAGEN), psiX174, pBluescript SA, pNH8A, pMH16A, pMH18A, pMH46A, pWLNEO, pSV2CAT, pOG44, pXTI et pSG (Stratagene).

De manière préférée, un vecteur d'expression d'un polypeptide de fusion inhibiteur spécifique tel que défini précédemment est le vecteur pMS2CP-PEP58X déposé à la Collection Nationale de Cultures de Microorganismes le 8 Juillet 2003 sous le numéro d'accès 1-3067..

Un vecteur préféré comprenant l'acide nucléique comprenant (i) au moins une copie d'une séquence nucléotidique cible de la protéine de liaison à l'ARN contenue dans le polypeptide de fusion inhibiteur spécifique et (ii) le polynucléotide d'intérêt dont le contrôle de la traduction est recherché est le vecteur pRLucLuc-CMVin + 3'UTRGb (MS2) n, qui est décrit dans les exemples.

Les vecteurs d'expression eucaryote préférés sont ceux décrits dans Makrides et al. (1999).

## ***CELLULES HOTES TRANSFORMEES SELON L'INVENTION.***

En général, un polypeptide de fusion inhibiteur spécifique de la traduction des protéines selon l'invention est utilisé pour inhiber spécifiquement la traduction d'une ou plusieurs protéines d'intérêt dans des cellules. Pour la mise en oeuvre des systèmes de contrôle de la traduction tels que définis ci-dessus, il est donc nécessaire de transfecter au préalable les cellules cibles avec un acide nucléique, ou un vecteur recombinant, permettant l'expression, le cas échéant inductible, dudit polypeptide de fusion dans ces cellules cibles.

La présente invention a donc également pour objet une cellule hôte transformée par un acide nucléique codant un polypeptide de fusion inhibiteur spécifique selon l'invention, ou par un vecteur recombinant dans lequel est inséré un tel acide nucléique.

La cellule hôte transformée peut être d'origine bactérienne, fongique ou encore toutes autres cellules eucaryotes.

Toutefois, de manière tout à fait préférée, la cellule hôte est une cellule de mammifère, y compris une cellule humaine.

De même, font partie de l'invention des cellules hôtes procaryotes ou eucaryotes comprenant un système de contrôle de la traduction des protéines telles que définies précédemment, à savoir :

- un acide nucléique codant un polypeptide de fusion inhibiteur spécifique de la traduction des protéines selon l'invention ;
- un acide nucléique comprenant le polynucléotide d'intérêt dont le contrôle de la traduction est recherché, et comprenant également le site nucléotidique cible de la protéine de liaison ARN contenue dans le polypeptide de fusion.

Dans certains cas, on cherchera tout d'abord à intégrer le polynucléotide cible du polypeptide de fusion inhibiteur spécifique considéré dans le génome de la cellule hôte procaryote ou eucaryote transformée, de manière à obtenir un système cellulaire stable de contrôle de la traduction de la ou des protéines d'intérêt cible.

Dans ce mode de réalisation particulier, le système de contrôle tel que défini précédemment est caractérisé en ce que l'acide nucléique (b) est inséré dans le génome de la cellule hôte procaryote ou eucaryote.

## **PROCEDES ET KITS SELON L'INVENTION**

L'invention a encore pour objet un procédé pour contrôler *in vitro* la traduction d'un polynucléotide cible d'intérêt, caractérisé en ce qu'il comprend les étapes suivantes :

a) on introduit dans une cellule hôte procaryote ou eucaryote un acide nucléique comprenant :

(i) au moins une copie d'une séquence nucléotidique cible d'une protéine de liaison à l'ARN ;
(ii) le polynucléotide d'intérêt ; et
(iii) un polynucléotide régulateur sous le contrôle duquel est placé ledit polynucléotide d'intérêt.

b) on cultive la cellule hôte recombinante obtenue à la fin de l'étape a) dans un milieu de culture approprié, la cellule hôte recombinante exprimant ledit polynucléotide d'intérêt;
c) lorsque désiré, on inhibe l'expression dudit polynucléotide d'intérêt en ajoutant au milieu de culture cellulaire un polypeptide de fusion inhibiteur spécifique tel que défini dans la présente description.

L'invention a également pour objet un procédé pour contrôler *in vitro* la traduction d'un polynucléotide cible d'intérêt, caractérisé en ce qu'il comprend les étapes suivantes :

a) on introduit dans une cellule hôte procaryote ou eucaryote

(1) un acide nucléique comprenant :

(i) au moins une copie d'une séquence nucléotidique cible d'une protéine de liaison à l'ARN ;
(ii) le polynucléotide d'intérêt ; et
(iii) un polynucléotide régulateur sous le contrôle duquel est placé ledit polynucléotide d'intérêt.

et
(2) un acide nucléique comprenant un polynucléotide codant un polypeptide de fusion inhibiteur spécifique selon l'invention placé sous le contrôle d'un polynucléotide régulateur inductible ;

b) on cultive la cellule hôte eucaryote ou procaryote dans un milieu de culture approprié ;
c) lorsque désiré, on ajoute au milieu de culture une concentration finale appropriée d'un agent permettant l'activation ou la répression de l'expression du polynucléotide codant le polypeptide de fusion.

En référence au tableau 2, ainsi qu'aux figures 4 et 5, l'agent d'activation peut consister :

- en la tétracycline, lorsque le polynucléotide régulateur inductible comprend la séquence TetO,
- en la tétracycline, lorsque le polynucléotide régulateur inductible comprend la séquence Tet P.

L'invention a également pour objet un kit ou trousse pour le contrôle de la traduction d'un polynucléotide d'intérêt, caractérisé en ce qu'il comprend un polypeptide de fusion inhibiteur spécifique tel que défini précédemment.
L'invention concerne aussi un kit ou trousse pour le contrôle de la traduction d'un polynucléotide d'intérêt, caractérisé en ce qu'il comprend :

(a) un polypeptide de fusion inhibiteur spécifique tel que défini précédemment ; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt

L'invention concerne aussi un kit ou trousse pour le contrôle de la traduction d'un polynucléotide d'intérêt, caractérisé en ce qu'il comprend un vecteur recombinant dans lequel est inséré un acide nucléique comprenant un polynucléotide codant un polypeptide de fusion inhibiteur spécifique, de préférence placé sous le contrôle d'un polynucléotide régulateur, de manière tout à fait préférée sous le contrôle d'un polynucléotide régulateur inductible.
L'invention est également relative à un kit ou trousse pour le contrôle
d'intérêt, caractérisé en ce qu'il comprend :

(a) un vecteur recombinant dans lequel est inséré un acide nucléique comprenant un polynucléotide codant un polypeptide de fusion inhibiteur spécifique selon l'invention , éventuellement placé sous le contrôle d'un polynucléotide régulateur, de manière tout à fait préférée placé sous le contrôle d'un polynucléotide régulateur inductible; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt

Selon un premier aspect avantageux, le kit ou trousse tel que défini ci-dessus est caractérisé en ce que le vecteur recombinant (a) est le vecteur recombinant (a) est le vecteur Pms2cp-PEP58X déposé à la Collection Nationale de Cultures de Microorganismes le 8 Juillet 2003 sous le numéro d'accès I-3067.
L'invention est également relative à l'utilisation d'un système de contrôle de la traduction des protéines telles que définies dans la présente description, ou d'un kit tel que défini ci-dessus pour le contrôle de la traduction d'un polynucléotide d'intérêt.

Selon un premier aspect, l'utilisation ci-dessus est caractérisée en ce que le polynucléotide d'intérêt est exprimé dans un système acellulaire.
Selon un second aspect avantageux l'utilisation ci-dessus est caractérisée en ce que le polynucléotide d'intérêt est exprimé *in vitro* par des cellules cultivées dans un bioréacteur.

## COMPOSITIONS PHARMACEUTIQUES SELON L'INVENTION

L'invention a également pour objet une composition pharmaceutique comprenant un polypeptide de fusion inhibiteur spécifique de la traduction des protéines tel que défini dans la présente description.
L'invention est également relative à une composition pharmaceutique comprenant :

(a) un polypeptide de fusion inhibiteur spécifique de la traduction des protéines telles que définies dans la présente description ; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt.

L'invention concerne aussi une composition pharmaceutique comprenant un vecteur recombinant dans lequel est inséré un acide nucléique comprenant un polynucléotide codant un polypeptide de fusion inhibiteur spécifique de la traduction des protéines selon l'invention, le cas échéant placé sous le contrôle d'un polynucléotide régulateur, de préférence un polynucléotide régulateur inductible.
L'invention a également trait à une composition pharmaceutique comprenant :

(a) un vecteur recombinant dans lequel est inséré un acide nucléique codant un polypeptide de fusion inhibiteur spécifique de la traduction des protéines tel que défini dans la présente description, éventuellement placé sous le contrôle d'un polynucléotide régulateur, de préférence un polynucléotide régulateur inductible; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt.

En thérapie cellulaire, les systèmes de contrôle de la traduction des protéines selon l'invention remplacent avantageusement le couple régulateur-inducteur utilisé actuellement par une molécule unique, le polypeptide de fusion inhibiteur spécifique tel que défini précédemment, qui est capable de moduler directement et très spécifiquement l'expression d'un gène codant pour une protéine d'intérêt thérapeutique. Avec les systèmes de contrôle selon l'invention, la régulation de l'expression du ou des gènes d'intérêt se réalise de manière post-transcriptionnelle, dans le cytoplasme des cellules hôtes, ce qui permet une action rapide et réversible de l'inducteur.
Pour une application des systèmes de contrôle selon l'invention à la correction directe de certains défauts d'expression de gènes cellulaires (thérapie « régulomique »), le polypeptide de fusion inhibiteur spécifique contiendra une protéine se fixant spécifiquement sur l'ARN messager cellulaire cible. Une spécificité cellulaire de ce type de traitement peut de plus être obtenue si l'ARN messager cible n'est exprimé que dans un type cellulaire donné, et/ou si le polypeptide de fusion inhibiteur spécifique utilisé n'interfère qu'avec des régulateurs spécifiques de ce type cellulaire.
En thérapie antivirale ou antiparasitaire, les drogues utilisées actuellement ont souvent des effets collatéraux indésirables liés à un manque de spécificité d'action. Parmi les cibles de ces drogues, on trouve des protéines cellulaires comme les polymérases. Au contraire, un polypeptide de fusion inhibiteur spécifique selon l'invention est susceptible d'agir directement et spécifiquement sur les ARN messagers issus du génome viral et non pas sur les produits codés par le génome de la cellule infectée. En effet, la protéine de liaison à l'ARN constitutive du polypeptide de fusion inhibiteur spécifique de l'invention est sélectionnée sur la base de sa spécificité pour l'ARN messager viral que l'on souhaite neutraliser. Cette stratégie permet d'obtenir une très bonne spécificité de ciblage du polypeptide de fusion inhibiteur spécifique de l'invention. De plus, étant entendu que l'expression des protéines virales est régulée pour une très large part au niveau post-transcriptionnel, un polypeptide de fusion inhibiteur spécifique selon l'invention est particulièrement bien adapté à la lutte antivirale.
De même, comme déjà mentionné, dans le domaine de la production des protéines d'intérêt en bioréacteur, le problème récurrent de la toxicité pour les cellules de la protéine d'intérêt dont on cherche l'expression peut être surmonté en réprimant l'expression du gène d'intérêt, grâce aux polypeptides de fusion inhibiteur spécifique de l'invention, pendant la phase de croissance des cellules, puis on active le gène codant la protéine d'intérêt à produire pendant les phases ultérieures, par exemple une fois que les cellules ont atteint une phase de croissance en plateau. En outre, en agissant sur la concentration du polypeptide de fusion inhibiteur spécifique présent dans le milieu réactionnel, il est vraisemblablement possible de contrôler très finement l'expression du gène d'intérêt pendant toutes les étapes du processus de production bioréacteur.

La présente invention est en outre illustrée, sans pour autant être limitée, par les exemples suivants.

## EXEMPLES

### EXEMPLE 1 - Protocole de construction des vecteurs recombinants MS2CP-Pep58X et MS2CP-Pep58H.

Les amplimères correspondant à Pep58X et Pep58H obtenus en utilisant comme matrice respectivement le plasmide pT7TSEDENBP (oligonucléotides ATGCTAGCGTAAAGTTCGCAGACACTCAGAAAG [SEQ ID N 12] et ATGCGGCCGCTGCATTGAGCTGCTGCATTTGC) [SEQ ID N°13] et le plasmide pT7TSCUGBP (oligonucléotides ATGCTAGCGTAAAATTTGCTGATACACAGAAG [SEQ ID N°14] et ATGCGGCCGCTGCGCTGATTTGCTGCATCTGC [SEQ ID N°15]) (Paillard, 2002) sont digérés par Nhel et Xho I et insérés dans le vecteur pMS2CP-HA préalablement digéré par Nhel et Xhol. Le vecteur pMS2CP-HA a été obtenu par insertion du tag HA (TACCCATACGATGTTCCAGATTACGCT [SEQ ID N°16]) dans le vecteur pcNMS2 (Lykke Andersen, Cell (103) 1121-31).

### EXEMPLE 2: Protocole de construction du vecteur pRLucLuc-CMVin + 3' UTRGb (MS2)n

Le plasmide pRL-Null (Promega) digéré Xbal est lié aux oligos hybridés (et) pour obtenir le plasmide pRLuc-Xbl. Le plasmide pGL3-Basic (Promega) est digéré BamHI/HindIII pour libérer l'insert SV40-Luc+ qui est sous cloné dans le vecteur pRL-Xbl digéré par BamHI/HindIII pour obtenir le plasmide pRLuc-Luc.

Un fragment comportant le promoteur CMV bidirectionnel et les introns b-globine et EF-1 (Généthon, Evry) est inséré dans les sites XbaI/MluI de pRLucLuc pour obtenir le plasmide pRLucLucCMVin. Le fragment comportant la 3' non traduite b globine et 8 répétitions MS2 obtenu par digestion Notl/Apal du plasmide pGB(8MS2) (Lykke Andersen, Cell (103) 1121-31) et inséré dans pRLucLuc linéarisé par Pvull pour obtenir le plasmide pRLucLuc-CMVin+3'UTRgb $(MS2)_8$.

### EXEMPLE 3: Caractérisation de l'activité d'un peptide inhibiteur selon l'invention.

#### A. Matériel et Méthodes

On a testé l'activité inhibitrice des peptides Pep60X et Pep61X de 17 et 28 acides aminés, qui correspondent respectivement aux acides aminés 211-227 (Pep60X) et 222-238 (Pep61X) de la séquence de la protéine EDENBP.

Les ARNm utilisés sont préparés à partir des constructions décrites dans Ezzedine et al., 2001. Une femtomole d'ARNm CAT-Eg2Delta2, qui ne comporte pas de séquence EDEN dans sa 3' non traduite, est co-injecté avec 200 ng de peptide dans des embryons de Xénope au stade 2 cellules.

Après 4,5 heures d'incubation, cinq lots de trois embryons sont collectés pour chaque série d'injection et des extraits protéiques sont préparés afin de mesurer l'activité CAT.

#### B. Résultats

Les résultats correspondant sont présentés sur la Figure 6.

La moyenne et l'écart type des mesures réalisées sont présentés figure 7A. Seul le peptide Pep58X démontre une activité d'inhibition significative de l'expression de l'ARNm CAT-Eg2Delta2. L'expérience a été reproduite avec des ARNm comportant une séquence EDEN, produits à partir du plasmide pEg2-Delta1. La co-injection de Pep58X induit une répression traductionnelle analogue pour les ARNm pEg2-Delta1 et pEg2-Delta2 (Fig 6B).

### EXEMPLE 4 : Caractérisation fonctionnelle du polypeptide de fusion inhibiteur spécifique MS2CP-Pep58X dans des cellules de mammifères *ex vivo.*

#### A. Matériel et Méthodes

Les cellules sont cotransfectées avec l'un des vecteurs de la série pMS2CP et le vecteur rapporteur.

Les niveaux de traduction de la luciférase Renillia (R) et de la luciférase Firefly (F) sont déterminés par mesure de luminescence (Promega's Dual Luciferase® Assay System).

La quantité de protéines de fusion produite est évaluée par Western blot (anticorps polyclonal anti HA, Santa Cruz Biotechnology), et normalisée par rapport à la quantité d'une protéine cellulaire ubiquitaire, la protéine PCNA (anticorps monoclonal Anti-Proliferating Cell Nuclear Antigen (PCNA), Sigma Aldrich Company). L'ARNm Luc F ne comporte pas de site MS2 dans sa partie 3' non traduite et ne devrait pas être affecté par l'expression de MS2CP-Pep58X. Pour le vérifier, l'expression de Luc F en présence de MS2CP-Pep58X est analysé par le calcul de d(LucF)/d (concentration en MS2CP-Pep58X).

Les résultats sont présentés Fig 7A. La pente de la droite obtenue est proche de un (0,998) vérifiant que la protéine de fusion produite n'affecte pas l'expression de la luciférase F. L'expression de Luc F a été ensuite utilisé comme standard interne pour estimer l'effet de la protéine de fusion sur la traduction de l'ARNm Luc R. Ce dernier comporte en effet dans sa partie 3' non traduite les sites MS2 permettant la fixation de la protéine MS2CP-Pep58X.

Les résultats sont analysés par le calcul de d(R/F) /d(concentration en MS2CP-Pep58X) (Fig 7B). L'expérience a été réalisé en parallèle avec MS2CP, les valeurs obtenues avec MS2CP correspondent à cent pour cent d'expression de Luc R.

## B. Résultats

Les résultats sont présentés sur la Figure 7, qui illustre les résultats de la traduction de la protéine marqueur luciférase en mettant en oeuvre un système de contrôle de la traduction protéique incluant le vecteurs pMS2CP-Pep58X, pMS2CP (utilisé comme Témoin), et le vecteur rapporteur pRLuc Luc CMVin + 3'UTRgb $(MS2)_8$.

L'expression de MS2CP-Pep58X provoque une répression de la traduction de l'ARNm Luc R, la pente de la droite correspondante est d'environ - 0,6 (Fig 7B). Le demandeur a montré que le système d'analyse permet également l'étude de protéines qui stimulent la traduction (résultats non présentés).

**Tableau 2 : Systèmes de régulation inductible préférés**

| NOM | PROMOTEUR | INDUCTEUR | REFERENCE BIBLIOGRAPHIQUES OU COMMERCIALE |
|---|---|---|---|
| pMSG | MMTV-LTR "(mouse mammary tumor virus") | Dexamethasone | Amersham Pharmacia |
| pOPRSVI/MCS | RSV-LTR (« Rous sarcoma virus ») | IPTG | Stratagene |
| pTet-Splice | Tet | Tetracycline | Life Technologies |
| pTRE | hCMV-1 | Tetracycline ou doxycycline | Clontech |
| pRev-TRE | hCMV-1 | Tetracycline ou doxycycline | Clontech |
| [2]pRetro-On pRetro-Off | hCMV-1 | Tetracycline ou doxycycline | Clontech |
| pIND series | ΔHSP ("Heat shock protein") | Ecdysone | Invitrogen |
| pPOP | mPGK/lacO (phosphoglycérate kinase) | IPTG | G. N. Hannan, S. A. Lehnert, E. S. MacAvoy, P. A. Jennings and P. L. Molloy, An engineered *PGK* promoter and *lac* operator-repressor system for the regulation of gene expression in mammalian cells. *Gene* **130** (1993), pp. 233-239. |

(suite)

| NOM | PROMOTEUR | INDUCTEUR | REFERENCE BIBLIOGRAPHIQUES OU COMMERCIALE |
|---|---|---|---|
| pEF-LAC | hEF-1$\alpha$/lacO | IPTG | Edamatsu, H., Kaziro, Y., and Itoh,H. (1997) Inductible high level expression vector for mammalian cells, pEF-LAC carrying human elongation factor 1 $\alpha$ promoter and lac operator. *Gene* **187**, 289-294. |
| pBPVMT1 | mMT-I (metallothionein I) | $Cd^{++}$ ,$Zn^{++}$, $PMA^{++}$ | Pavlakis, G.N., and Hamer, D.H.(1983) Regulation of a metallothionein-growth hormone hybrid gene in bovine papilloma *virus.Proc. Natl. Acad Sci. USA* **80,** 397-401 |
| pMT | hMT-II (metallothionein II) | $Cd^{++}$, $Zn^{++}$, $PMA^{++}$ | Friedman, J.S., Cofer,C.L., Anderson, C.L., Kushner, J.A., Gray, P.P., Chapman, G.E., Stuart, M.C., Lazarus, L., Shine, J., and Kushner, P.J. (1989) High expression in mammalian cells without amplification. *Biol Technology* **7**,359-362 |
| pMT302 | hMT-IIA(mutant) | $Cd^{++}$, $Zn^{++}$, $PMA^{++}$ | Makarov, S.S., Jonat,C., and Haskill, S. (1994) Hyperinductible human metallothionein promoter with a low level basal activity. *Nucleic Acids Res.* **22** 1504-1505 |
| pIPF | hIFN-$\alpha$, (interferon $\alpha$) | Virus | Mori,T., Yamamoto, K., Ohta, T., Sakamoto, C., Sato, M., Koide, K., Murakami, T., Fujii, M., Fukuda, S., and Kurimoto, M. (1994) A high level and regulatable production system for recombinant glycoproteins using a human interferon-$\alpha$ promoter-based expression vector. *Gene* **144** 289-293 |
| pGRE5 | 5XGRE/Ad2MLP "(glucocorticoid reponse element/adenovirus major late promoter") | Dexamethasone | Mader,S., and White, J.H. (1993) A steroid-inductible promoter for the controlled overexpression of cloned genes in eukaryotic cells. |

(suite)

| NOM | PROMOTEUR | INDUCTEUR | REFERENCE BIBLIOGRAPHIQUES OU COMMERCIALE |
|---|---|---|---|
| GRE5 | "high affinity glucocorticoid reponse element (GRE)/ Adenovirus 2MLP" | Dexamethasone | S. Mader and J. H. White, A steroid-inducible promoter for the controlled overexpression of cloned genes in eukaryotic cells. *Proc. Natl. Acad. Sci. USA* **90** (1993), pp. 5603-5607 0 (12) 5603-7 |
| pRDB | "DRE/MMTV (dioxin reponse element)" | TTCD' | . A. De Benedetti and R. E. Rhoads , A novel BK virus-based episomal vector for expression of foreign genes in mammalian cells. *Nucleic Acids Res.* **19** (1991), pp. 1925-1931. |

**TABLEAU 3**

| SEQ ID N° | Désignation | Type |
|---|---|---|
| 1 | Pep58X aa | peptide |
| 2 | Pep 58H aa | peptide |
| 3 | EDEN-BP (aa84-155) | peptide |
| 4 | CUG-BP (aa155-242) | peptide |
| 5 | Fusion MS2CP-HA TAG-Pep58X | peptide |
| 6 | Fusion MS2CP-HA TAG-Pep 58H | peptide |
| 7 | Fusion MS2-CP-HA TAG-Pep58X | acide nucléique |
| 8 | Fusion MS2CP-HA TAG-Pep 58H | acide nucléique |
| 9 | EDENBP | peptide |
| 10 | EDENBP | acide nucléique |
| 11 | Peptide HA | peptide |
| 12 | Amorce | acide nucléique |
| 13 | Amorce | acide nucléique |
| 14 | Amorce | acide nucléique |
| 15 | Amorce | acide nucléique |
| 16 | Peptide HA | acide nucléique |

**REFERENCES**

- AUSUBEL et T. al., (1989) Current Protocols in Molecular Biology, Green Publishing Associates and Wiley Interscience N.Y.
- COLLER J. et al., 2002, Methods, vo1.26 : 142-150.
- EZZEDINE et al., 2002, PROC. Natl. Acad. Sci. USA, vol.99(1): 257-262.

- HOUBEN WEIL (1974); In methode der Organischen Chemie, E. Wunsh ed., volume 15-I et 15-II, Thieme, Stuttgart.
- MAKRIDES, Savvas C., 1999, Protein Expr. Purif., vol. 17(2):183-202.
- MERRIFIELD RB, (1965a), Nature, vol.207 (996): 522-523
- MERRIFIELD RB, (1965b), Science, vol.150 (693):178-185.
- PAILLARD L et al., 1998, The Embo Journal, vol.17 (1) 278-287.
- PAILLARD L et al., 2002, vol.277 (5) 3232-3235.
- UCHIDA N AOYUKI, Shin-ichi Hoshino, Hiroaki Imataka, Nahum Sonenberg and Toshiaki Katada.J. Biol. Chem., Dec. 2002 ; 277 :50286-50292.


**Revendications**

1. Peptide inhibiteur de la traduction des protéines, **caractérisé en ce que** sa taille est d'au plus 250 acides aminés et **en ce qu'**il comprend une séquence d'acides aminés possédant au moins 85% d'identité avec la séquence en acides aminés SEQ ID N°1.

2. Peptide selon la revendication 1, **caractérisé en ce que** sa taille est d'au plus 50 acides aminés.

3. Peptide selon la revendication 1, **caractérisé en ce que** sa taille est d'au plus 30 acides aminés, de préférence d'au plus 28 acides aminés.

4. Peptide selon l'une des revendications 1 à 3, **caractérisé en ce qu'**il comprend une séquence d'acides aminés possédant au moins 89% d'identité avec la séquence en acides aminés SEQ ID N°1.

5. Peptide selon l'une des revendications 1 à 4, **caractérisé en ce qu'**il comprend la séquence SEQ ID N°1.

6. Peptide selon l'une des revendications 1 à 4, **caractérisé en ce qu'**il comprend la séquence SEQ ID N°2.

7. Polypeptide de fusion inhibant spécifiquement la traduction d'un polynucléotide cible d'intérêt, **caractérisé en ce que** ledit polypeptide comprend un peptide selon l'une des revendications 1 à 6, ledit peptide étant fusionné avec une protéine de liaison à l'ARN.

8. Polypeptide de fusion selon la revendication 7, **caractérisé en ce que** la protéine de liaison à l'ARN est choisie parmi MS2CP, N, IRP ET U1A.

9. Acide nucléique comprenant un polynucléotide codant un peptide selon l'une des revendications 1 à 6.

10. Acide nucléique selon la revendication 9, **caractérisé en ce qu'**il comprend un polynucléotide régulateur sous le contrôle duquel est placé le polynucléotide codant le peptide.

11. Acide nucléique comprenant un polynucléotide codant un polypeptide de fusion selon l'une des revendications 7 et 8.

12. Acide nucléique selon la revendication 11, **caractérisé en ce qu'**il comprend un polynucléotide régulateur sous le contrôle duquel est placé le polynucléotide codant le polypeptide de fusion.

13. Acide nucléique selon la revendication 12, **caractérisé en ce que** le polynucléotide régulateur est un polynucléotide régulateur inductible.

14. Système de contrôle de la traduction d'un polynucléotide cible d'intérêt comprenant un polypeptide de fusion selon l'une des revendications 7 ou 8, ou un acide nucléique selon l'une des revendications 11 à 13.

15. Système de contrôle de la traduction d'un polynucléotide cible d'intérêt comprenant :

    (a) un premier acide nucléique consistant en un acide nucléique selon l'une des revendications 11 à 13 ;
    (b) un second acide nucléique comprenant :

        (i) au moins une copie d'une séquence nucléotidique cible de la protéine de liaison à l'ARN contenue dans le polypeptide de fusion codé par le premier acide nucléique tel que défini en (a) ;
        (ii) le polynucléotide d'intérêt.

16. Système de contrôle de la traduction d'un polynucléotide cible d'intérêt comprenant :

(a) un polypeptide de fusion selon l'une des revendications 7 ou 8;
(b) un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt.

**17.** Système de contrôle selon l'une des revendications 15 ou 16, **caractérisé en ce que** le second acide nucléique comprend un polynucléotide régulateur sous le contrôle duquel est placé le polynucléotide d'intérêt.

**18.** Acide nucléique selon l'une des revendications 9 et 10, **caractérisé en ce qu'**il est inséré dans un vecteur recombinant de clonage ou d'expression.

**19.** Vecteur de clonage ou d'expression recombinant comprenant un acide nucléique selon l'une des revendications 9 et 10.

**20.** Acide nucléique selon l'une des revendications 11 à 13, **caractérisé en ce qu'**il est inséré dans un vecteur recombinant de clonage ou d'expression.

**21.** Vecteur de clonage ou d'expression recombinant comprenant un acide nucléique selon l'une des revendications 11 à 13.

**22.** Vecteur selon la revendication 21, **caractérisé en ce qu'**il s'agit du vecteur pMS2CP-PEP58X déposé à la Collection Nationale de Cultures de Microorganismes le 8 Juillet 2003 sous le numéro d'accès I-3067.

**23.** Système de contrôle selon l'une des revendications 15 à 17, **caractérisé en ce que** le ou les acides nucléiques qui sont inclus dans celui-ci sont insérés dans un vecteur d'expression recombinant.

**24.** Système de contrôle selon l'une des revendications 15 à 17, **caractérisé en ce que** l'acide nucléique (b) est inséré dans le génome d'une cellule hôte procaryote ou eucaryote.

**25.** Cellule hôte procaryote ou eucaryote comprenant un système de contrôle selon l'une des revendications 15 à 17, 23 et 24.

**26.** Cellule hôte procaryote ou eucaryote comprenant un acide nucléique selon l'une des revendications 11 à 13 ou un vecteur recombinant selon la revendication 21.

**27.** Procédé pour contrôler *in vitro* la traduction d'un polynucléotide cible d'intérêt, **caractérisé en ce qu'**il comprend les étapes suivantes :

a) on introduit dans une cellule hôte procaryote ou eucaryote un acide nucléique comprenant :

(i) au moins une copie d'une séquence nucléotidique cible d'une protéine de liaison à l'ARN ;
(ii) le polynucléotide d'intérêt ; et
(iii) un polynucléotide régulateur sous le contrôle duquel est placé ledit polynucléotide d'intérêt.

b) on cultive la cellule hôte recombinante obtenue à la fin de l'étape a) dans un milieu de culture approprié, la cellule hôte recombinante exprimant ledit polynucléotide d'intérêt;
c) lorsque désiré, on inhibe l'expression dudit polynucléotide d'intérêt en ajoutant au milieu de culture cellulaire un polypeptide de fusion selon l'une des revendications 7 ou 8.

**28.** Procédé pour contrôler *in vitro* la traduction d'un polynucléotide cible d'intérêt, **caractérisé en ce qu'**il comprend les étapes suivantes :

a) on introduit dans une cellule hôte procaryote ou eucaryote

(1) un acide nucléique comprenant :

(i) au moins une copie d'une séquence nucléotidique cible d'une protéine de liaison à l'ARN ;
(ii) le polynucléotide d'intérêt ; et
(iii) un polynucléotide régulateur sous le contrôle duquel est placé ledit polynucléotide d'intérêt.

et
(2) un acide nucléique selon la revendication 13;.

b) on cultive la cellule hôte eucaryote ou procaryote dans un milieu de culture approprié ;
c) Lorsque désiré, on ajoute au milieu de culture une concentration finale appropriée d'un agent permettant l'activation ou la répression de l'expression du polynucléotide codant le polypeptide de fusion.

**29.** Kit ou trousse pour le contrôle de la traduction d'un polynucléotide d'intérêt, **caractérisé en ce qu'**il comprend un polypeptide de fusion selon l'une des revendications 7 ou 8.

**30.** Kit ou trousse pour le contrôle de la traduction d'un polynucléotide d'intérêt, **caractérisé en ce qu'**il comprend :

(a) un polypeptide de fusion selon l'une des revendications 7 ou 8; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt

**31.** Kit ou trousse pour le contrôle de la traduction d'un polynucléotide d'intérêt, **caractérisé en ce qu'**il comprend un vecteur recombinant dans lequel est inséré un acide nucléique selon l'une des revendications 11 à 13.

**32.** Kit ou trousse pour le contrôle de la traduction d'un polynucléotide d'intérêt, **caractérisé en ce qu'**il comprend :

(a) un vecteur recombinant dans lequel est inséré un acide nucléique selon l'une des revendications 11 à 13; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt

**33.** Kit ou trousse selon la revendication 32, **caractérisé en ce que** le vecteur recombinant (a) est le vecteur pMS2CP-PEP58X déposé à la Collection Nationale de Cultures de Microorganismes le 8 Juillet 2003 sous le numéro d'accès 1-3067.

**34.** Utilisation d'un système de contrôle selon l'une des revendications 15 à 17, 23 et 24 ou d'un kit selon l'une des revendications 29 à 33 pour le contrôle in vitro de la traduction d'un polynucléotide d'intérêt.

**35.** Utilisation selon la revendication 34, **caractérisée en ce que** le polynucléotide d'intérêt est exprimé dans un système acellulaire.

**36.** Utilisation selon la revendication 34, **caractérisée en ce que** le polynucléotide d'intérêt est exprimé *in vitro* par des cellules cultivées dans un bioréacteur.

**37.** Composition pharmaceutique comprenant un polypeptide de fusion selon l'une des revendications 7 ou 8.

**38.** Composition pharmaceutique comprenant :

(a) un polypeptide de fusion selon l'une des revendications 7 ou 8; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt

**39.** Composition pharmaceutique comprenant un vecteur recombinant dans lequel est inséré un acide nucléique selon l'une des revendications 11 à 13.

**40.** Composition pharmaceutique comprenant :

(a) un vecteur recombinant dans lequel est inséré un acide nucléique selon l'une des revendications 11 à 13; et
(b) un vecteur recombinant dans lequel est inséré un acide nucléique comprenant :

(i) au moins une copie d'une séquence nucléotidique cible de la protéine de liaison à l'ARN contenue dans le polypeptide de fusion tel que défini en (a) ;
(ii) le polynucléotide d'intérêt

**Claims**

**1.** Inhibitor peptide of the translation of proteins, **characterised in that** its length is up to 250 amino acids and **in that** it comprises an amino acid sequence possessing at least 85% identity with the amino acid sequence SEQ ID N° 1.

**2.** Peptide according to Claim 1, **characterised in that** its length is up to 50 amino acids.

**3.** Peptide according to Claim 1, **characterised in that** its length is up to 30 amino acids, preferably up to 28 amino acids.

**4.** Peptide according to one of the Claims 1 to 3, **characterised in that** it comprises an amino acid sequence having at least 89% identity with the amino acid sequence SEQ ID N° 1.

**5.** Peptide according to one of the Claims 1 to 4, **characterised in that** it comprises the sequence SEQ ID N° 1

**6.** Peptide according to one of the Claims 1 to 4, **characterised in that** it comprises the sequence SEQ ID N° 2.

**7.** Fusion polypeptide specifically inhibiting the translation of a target polynucleotide of interest, **characterised in that** said polypeptide comprises a peptide according to one of the Claims 1 to 6, said peptide being fused with an RNA binding protein.

**8.** Fusion polypeptide according to Claim 7, **characterised in that** the RNA binding protein is selected from MS2CP, N, IRP and U1A.

**9.** Nucleic acid comprising a polynucleotide coding for a peptide according to one of the Claims 1 to 6.

**10.** Nucleic acid according to Claim 9, **characterised in that** it comprises a regulatory polynucleotide under the control of which is placed the polynucleotide coding for the peptide.

**11.** Nucleic acid comprising a polynucleotide coding for a fusion polypeptide according to one of the Claims 7 and 8.

**12.** Nucleic acid according to Claim 11, **characterised in that** it comprises a regulatory polynucleotide under the control of which is placed the polynucleotide coding for the fusion polypeptide.

**13.** Nucleic acid according to Claim 12, **characterised in that** the regulatory polynucleotide is an inducible regulatory polynucleotide.

**14.** Control system of the translation of a target polynucleotide of interest comprising a fusion polypeptide according to one of the Claims 7 or 8, or a nucleic acid according to one of the Claims 11 to 13.

**15.** Control system of the translation of a target polynucleotide of interest comprising:

(a) a first nucleic acid consisting of a nucleic acid according to one of the Claims 11 to 13;
(b) a second nucleic acid comprising:

(i) at least one copy of a target nucleotide sequence of the RNA binding protein contained in the fusion polypeptide encoded in the first nucleic acid such as defined in (a);
(ii) the polynucleotide of interest.

**16.** Control system of the translation of a target polynucleotide of interest comprising:

(a) a fusion polypeptide according to one of the Claims 7 or 8;
(b) a nucleic acid comprising:

(i) at least one copy of a target nucleotide sequence of the RNA binding protein contained in the fusion polypeptide such as defined in (a);
(ii) the polynucleotide of interest.

**17.** Control system according to one of the Claims 15 or 16, **characterised in that** the second nucleic acid comprises a regulatory polynucleotide under the control of which is placed the polynucleotide of interest.

**18.** Nucleic acid according to one of the Claims 9 and 10, **characterised in that** it is inserted into a recombinant cloning or expression vector.

**19.** Recombinant cloning or expression vector comprising a nucleic acid according to one of the Claims 9 and 10.

**20.** Nucleic acid according to one of the Claims 11 to 13, **characterised in that** it is inserted in a recombinant cloning or expression vector.

**21.** Recombinant cloning or expression vector comprising a nucleic acid according to one of the Claims 11 to 13.

**22.** Vector according to Claim 21, **characterised in that** it is the vector pMS2CP-PEP58X filed at the Collection Nationale de Cultures de Microorganismes on $8^{th}$ July 2003 under the access number I-3067.

**23.** Control system according to one of the Claims 15 to 17, **characterised in that** the nucleic acid(s) which is/are included in the latter is/are inserted in a recombinant expression vector.

**24.** Control system according to one of the Claims 15 to 17, **characterised in that** the nucleic acid (b) is inserted into the genome of a prokaryotic or eukaryotic host cell.

**25.** Prokaryotic or eukaryotic host cell comprising a control system according to one of the Claims 15 to 17, 23 and 24.

**26.** Prokaryotic or eukaryotic host cell comprising a nucleic acid according to one of the Claims 11 to 13 or a recombinant vector according to Claim 21.

**27.** Process for the *in vitro* control of the translation of a target polynucleotide of interest, **characterised in that** it comprises the following steps:

a) into a prokaryotic or eukaryotic host cell is introduced a nucleic acid comprising:

(i) at least one copy of a target nucleotide sequence of an RNA binding protein;
(ii) the polynucleotide of interest; and
(iii) a regulatory polynucleotide under the control of which is placed said polynucleotide of interest.

(b) the recombinant host cell obtained at the end of step a) is cultivated in a suitable culture medium, the recombinant host cell expressing the said polynucleotide of interest;
(c) when desired, the expression of the said polynucleotide of interest is inhibited by adding to the cell culture medium a fusion polypeptide according to one of the Claims 7 or 8.

**28.** Process for the *in vitro* control of the translation of a target polynucleotide of interest, **characterised in that** it comprises the following steps:

(a) into a prokaryotic or eukaryotic host cell are introduced:

(1) a nucleic acid comprising:

(i) at least one copy of a target nucleotide sequence of an RNA binding protein;
(ii) the polynucleotide of interest; and
(iii) a regulatory polynucleotide under the control of which is placed the said polynucleotide of interest.

and
(2) a nucleic acid according to Claim 13;

(b) the prokaryotic or eukaryotic host cell is cultivated in a suitable culture medium;
(c) when desired, a suitable final concentration of an agent permitting the activation or repression of the expression of the polynucleotide coding for the fusion polypeptide is added to the culture medium.

**29.** Kit for the control of the translation of a polynucleotide of interest, **characterised in that** it comprises a fusion polypeptide according to one of the Claims 7 or 8.

**30.** Kit for the control of the translation of a polynucleotide of interest, **characterised in that** it comprises:

(a) a fusion polypeptide according to one of the Claims 7 or 8; and
(b) a recombinant vector in which is inserted a nucleic acid comprising:

(i) at least one copy of a target nucleotide sequence of the RNA binding protein contained in the fusion polypeptide such as defined in (a);
(ii) the polynucleotide of interest.

**31.** Kit for the control of the translation of a polynucleotide of interest, **characterised in that** it comprises a recombinant vector in which is inserted a nucleic acid according to one of the Claims 11 to 13.

**32.** Kit for the control of the translation of a polynucleotide of interest, **characterised in that** it comprises:

(a) a recombinant vector in which is inserted a nucleic acid according to one of the Claims 11 to 13; and
(b) a recombinant vector in which is inserted a nucleic acid comprising:

(i) at least one copy of a target nucleotide sequence of the RNA binding protein contained in the fusion polypeptide such as defined in (a)
(ii) the polynucleotide of interest.

**33.** Kit according to Claim 32, **characterised in that** the recombinant vector (a) is the vector pMS2CP-PEP58X filed at the Collection Nationale de Cultures de Microorganismes on $8^{th}$ July 2003 under the access number I-3067.

**34.** Use of a control system according to one of the Claims 15 to 17, 23 and 24 or a kit according to one of the Claims 29 to 33 for the *in vitro* control of the translation of a polynucleotide of interest.

**35.** Use according to Claim 34, **characterised in that** the polynucleotide of interest is expressed in a cell-free system.

**36.** Use according to Claim 34, **characterised in that** the polynucleotide of interest is expressed *in vitro* by cells cultivated in a bioreactor.

**37.** Pharmaceutical composition comprising a fusion polypeptide according to one of the Claims 7 or 8.

**38.** Pharmaceutical composition comprising:

(a) a fusion polypeptide according to one of the Claims 7 or 8; and
(b)a recombinant vector in which is inserted a nucleic acid comprising:

(i) at least one copy of a target nucleotide sequence of the RNA binding protein contained in the fusion polypeptide such as defined in (a);
(ii) the polynucleotide of interest.

**39.** Pharmaceutical composition comprising a recombinant vector in which is inserted a nucleic acid according to one of the Claims 11 to 13.

**40.** Pharmaceutical composition comprising:

(a) a recombinant vector in which is inserted a nucleic acid according to one of the Claims 11 to 13; and
(b)a recombinant vector in which is inserted a nucleic acid comprising:

(i) at least one copy of a target nucleotide sequence of the RNA binding protein contained in the fusion polypeptide such as defined in (a)
(ii) the polynucleotide of interest.

**Patentansprüche**

**1.** Peptid-Inhibitor der Translation der Proteine, **dadurch gekennzeichnet, dass** seine Größe höchstens 250 Aminosäuren ist und dass er eine Aminosäuresequenz umfasst, die wenigstens 85% Identität mit der Aminosäuresequenz SEQ ID NO: 1 besitzt.

**2.** Peptid nach Anspruch 1, **dadurch gekennzeichnet, dass** seine Größe höchstens 50 Aminosäuren ist.

**3.** Peptid nach Anspruch 1, **dadurch gekennzeichnet, dass** seine Größe höchstens 30 Aminosäuren, vorzugsweise höchstens 28 Aminosäuren, ist.

**4.** Peptid nach einem der Ansprüche 1 bis 3, **dadurch gekennzeichnet, dass** es eine Aminosäuresequenz umfasst, die wenigstens 89% Identität mit der Aminosäuresequenz SEQ ID NO: 1 besitzt.

**5.** Peptid nach einem der Ansprüche 1 bis 4, **dadurch gekennzeichnet, dass** es die Sequenz SEQ ID NO: 1 umfasst.

**6.** Peptid nach einem der Ansprüche 1 bis 4, **dadurch gekennzeichnet, dass** es die Sequenz SEQ ID NO: 2 umfasst.

**7.** Fusionspolypeptid, das spezifisch die Translation eines Target-Polynucleotids von Interesse inhibiert, **dadurch gekennzeichnet, dass** das Polypeptid ein Peptid nach einem der Ansprüche 1 bis 6 umfasst, wobei das Peptid mit einem Bindungsprotein für RNA fusioniert ist.

**8.** Fusionspolypeptid nach Anspruch 7, **dadurch gekennzeichnet, dass** das Bindungsprotein für RNA unter MS2CP, N, IRP und U1A ausgewählt ist.

**9.** Nucleinsäure, die ein Polypeptid umfasst, das für ein Peptid nach einem der Ansprüche 1 bis 6 kodiert.

**10.** Nucleinsäure nach Anspruch 9, **dadurch gekennzeichnet, dass** sie ein Regulator-Polynucleotid umfasst, unter dessen Kontrolle das Polynucleotid, das für das Peptid kodiert, gestellt ist.

**11.** Nucleinsäure, die ein Polynucleotid umfasst, das für ein Fusionspolypeptid nach einem der Ansprüche 7 und 8 kodiert.

**12.** Nucleinsäure nach Anspruch 11, **dadurch gekennzeichnet, dass** sie ein Regulator-Polynucleotid umfasst, unter dessen Kontrolle das Polynucleotid, das für das Fusionspolypeptid kodiert, gestellt ist.

**13.** Nucleinsäure nach Anspruch 12, **dadurch gekennzeichnet, dass** das Regulator-Polynucleotid ein induzierbares Regulator-Polynucleotid ist.

**14.** Kontrollsystem für die Translation eines Target-Polynucleotids von Interesse, das ein Fusionspolypeptid nach einem der Ansprüche 7 und 8 oder eine Nucleinsäure nach einem der Ansprüche 11 bis 13 umfasst.

**15.** Kontrollsystem für die Translation eines Target-Polynucleotids von Interesse, umfassend:

(a) eine erste Nucleinsäure, die aus einer Nucleinsäure nach einem der Ansprüche 11 bis 13 besteht;
(b) eine zweite Nucleinsäure, umfassend:

(i) wenigstens eine Kopie einer Target-Nucleotidsequenz des Bindungsproteins für RNA, das in dem Fusionspolypeptid enthalten ist, das durch die erste Nucleinsäure, wie sie in (a) definiert ist, kodiert wird;
(ii) das Polynucleotid von Interesse.

**16.** Kontrollsystem für die Translation eines Target-Polynucleotids von Interesse, umfassend:

(a) ein Fusionspolypeptid nach einem der Ansprüche 7 und 8;
(b) eine Nucleinsäure, umfassend:

(i) wenigstens eine Kopie einer Target-Nucleotidsequenz des Bindungsproteins für RNA, das im Fusionspolypeptid enthalten ist, wie es in (a) definiert ist;
(ii) das Polynucleotid von Interesse.

**17.** Kontrollsystem nach einem der Ansprüche 15 und 16, **dadurch gekennzeichnet, dass** die zweite Nucleinsäure ein Regulator-Polynucleotid umfasst, unter dessen Kontrolle das Polynucleotid von Interesse gestellt ist.

**18.** Nucleinsäure nach einem der Ansprüche 9 und 10, **dadurch gekennzeichnet, dass** sie in einen rekombinanten Klonierungs- oder Expressionsvektor insertiert ist.

**19.** Rekombinanter Klonierungs- oder Expressionsvektor, der eine Nucleinsäure nach einem der Ansprüche 9 und 10 umfasst.

**20.** Nucleinsäure nach einem der Ansprüche 11 bis 13, **dadurch gekennzeichnet, dass** sie in einen rekombinanten Klonierungs- oder Expressionsvektor insertiert ist.

**21.** Rekombinanter Klonierungs- oder Expressionsvektor, der eine Nucleinsäure nach einem der Ansprüche 11 bis 13 umfasst.

**22.** Vektor nach Anspruch 21, **dadurch gekennzeichnet, dass** es sich um den Vektor pMS2CP-PEP58X handelt, der am 8. Juli 2003 bei der Collection Nationale de Cultures de Microorganismes unter der Eingangs-Nr. 1-3067 hinterlegt wurde.

**23.** Kontrollsystem nach einem der Ansprüche 15 bis 17, **dadurch gekennzeichnet, dass** die Nucleinsäure (n), die darin enthalten ist (sind), in einen rekombinanten Expressionsvektor insertiert ist (sind).

**24.** Kontrollsystem nach einem der Ansprüche 15 bis 17, **dadurch gekennzeichnet, dass** die Nucleinsäure (b) in das Genom einer prokaryotischen oder eukaryotischen Wirtszelle insertiert ist.

**25.** Prokaryotische oder eukaryotische Wirtszelle, die ein Kontrollsystem nach einem der Ansprüche 15 bis 17, 23 und 24 umfasst.

**26.** Prokaryotische oder eukaryotische Wirtszelle, die eine Nucleinsäure nach einem der Ansprüche 11 bis 13 oder einen rekombinanten Vektor nach Anspruch 21 umfasst.

**27.** Verfahren zur *in vitro*-Kontrolle der Translation eines Target-Polynucleotids von Interesse, **dadurch gekennzeichnet, dass** es die folgenden Stufen umfasst:

(a) in eine prokaryotische oder eukaryotische Wirtszelle führt man eine Nucleinsäure ein, die umfasst:

(i) wenigstens eine Kopie einer Target-Nucleotidsequenz eines Bindungsproteins für RNA;
(ii) das Polynucleotid von Interesse und
(iii) ein Regulator-Polynucleotid, unter dessen Kontrolle das Polynucleotid von Interesse gestellt ist;

(b) man kultiviert die rekombinante Wirtszelle, die am Ende der Stufe (a) erhalten wurde, in einem geeigneten Kulturmedium, wobei die rekombinante Wirtszelle das Polynukleotid von Interesse exprimiert;
(c) falls gewünscht, inhibiert man die Expression des Polynucleotids von Interesse, indem man dem Zellkulturmedium ein Fusionspolypeptid nach einem der Ansprüche 7 und 8 zusetzt.

**28.** Verfahren zur *in vitro*-Kontrolle der Translation eines Target-Polynucleotids von Interesse, **dadurch gekennzeichnet, dass** es die folgenden Stufen umfasst:

(a) man führt in eine prokaryotische oder eukaryotische Wirtszelle ein:

(1) eine Nucleinsäure, umfassend:

(i) wenigstens eine Kopie einer Target-Nucleotidsequenz eines Bindungsproteins für RNA;
(ii) das Polynucleotid von Interesse und
(iii) ein Regulator-Polynucleotid, unter dessen Kontrolle das Polynucleotid von Interesse gestellt ist,

(2) eine Nucleinsäure nach Anspruch 13;

(b) man kultiviert die eukaryotische oder prokaryotische Wirtszelle in einem geeigneten Kulturmedium;
(c) wenn gewünscht, setzt man dem Kulturmedium eine geeignete Endkonzentration eines Mittels zu, das die Aktivierung oder die Unterdrückung der Expression des Polynucleotids, das für das Fusionspolypeptid kodiert, ermöglicht.

**29.** Kit oder Set für die Kontrolle der Translation eines Polynucleotids von Interesse, **dadurch gekennzeichnet, dass** er/es ein Fusionspolypeptid nach einem der Ansprüche 7 und 8 umfasst.

**30.** Kit oder Set für die Kontrolle der Translation eines Polynucleotids von Interesse, **dadurch gekennzeichnet, dass** er/es umfasst:

(a) ein Fusionspolypeptid nach einem der Ansprüche 7 und 8 und
(b) einen rekombinanten Vektor, in den eine Nucleinsäure insertiert ist, die umfasst:

(i) wenigstens eine Kopie einer Target-Nucleotidsequenz des Bindungsproteins für RNA, das in dem Fusionspolypeptid enthalten ist, wie es in (a) definiert ist;
(ii) das Polynucleotid von Interesse.

**31.** Kit oder Set für die Kontrolle der Translation eines Polynucleotids von Interesse, **dadurch gekennzeichnet, dass** er/es einen rekombinanten Vektor umfasst, in den eine Nucleinsäure nach einem der Ansprüche 11 bis 13 insertiert ist.

**32.** Kit oder Set für die Kontrolle der Translation eines Polynucleotids von Interesse, **dadurch gekennzeichnet, dass** er/es umfasst:

(a) einen rekombinanten Vektor, in den eine Nucleinsäure nach einem der Ansprüche 11 bis 13 insertiert ist, und
(b) einen rekombinanten Vektor, in den eine Nucleinsäure insertiert ist, die umfasst:

(i) wenigstens eine Kopie einer Target-Nucleotidsequenz des Bindungsproteins für RNA, die in dem Fusionspolypeptid enthalten ist, wie es in (a) definiert ist;
(ii) das Polynucleotid von Interesse.

**33.** Kit oder Set nach Anspruch 32, **dadurch gekennzeichnet, dass** der rekombinante Vektor (a) der Vektor pMS2CP-PEP58X ist, der am 8. Juli 2003 bei der Collection Nationale de Cultures de Microorganismes unter der Eingangs-Nr. I-3067 hinterlegt wurde.

**34.** Verwendung eines Kontrollsystems nach einem der Ansprüche 15 bis 17, 23 und 24 oder eines Kits nach einem der Ansprüche 29 bis 33 für die *in vitro*-Kontrolle der Translation eines Polynucleotids von Interesse.

**35.** Verwendung nach Anspruch 34, **dadurch gekennzeichnet, dass** das Polynucleotid von Interesse in einem azellulären System exprimiert wird.

**36.** Verwendung nach Anspruch 34, **dadurch gekennzeichnet, dass** das Polynucleotid von Interesse *in vitro* durch Zellen exprimiert wird, die in einem Bioreaktor kultiviert werden.

**37.** Pharmazeutische Zusammensetzung, die ein Fusionspolypeptid nach einem der Ansprüche 7 und 8 umfasst.

**38.** Pharmazeutische Zusammensetzung, umfassend:

(a) ein Fusionspolypeptid nach Anspruch 7 oder 8; und
(b) einen rekombinanten Vektor, in den eine Nucleinsäure insertiert ist, die umfasst:

(i) wenigstens eine Kopie einer Target-Nnucleotidsequenz des Bindungsproteins für RNA, das im Fusionspolypeptid enthalten ist, wie es in (a) definiert ist;
(ii) das Polynucleotid von Interesse.

**39.** Pharmazeutische Zusammensetzung, die einen rekombinanten Vektor umfasst, in den eine Nucleinsäure nach einem der Ansprüche 11 bis 13 insertiert ist.

**40.** Pharmazeutische Zusammensetzung, umfassend:

(a) einen rekombinanten Vektor, in den eine Nucleinsäure nach einem der Ansprüche 11 bis 13 insertiert ist; und
(b) einen rekombinanten Vektor, in den eine Nucleinsäure insertiert ist, die umfasst:

(i) wenigstens eine Kopie einer Target-Nucleotidsequenz des Bindungsproteins für RNA, das im Fusionspolypeptid enthalten ist, wie es in (a) definiert ist;
(ii) das Polynucleotid von Interesse.

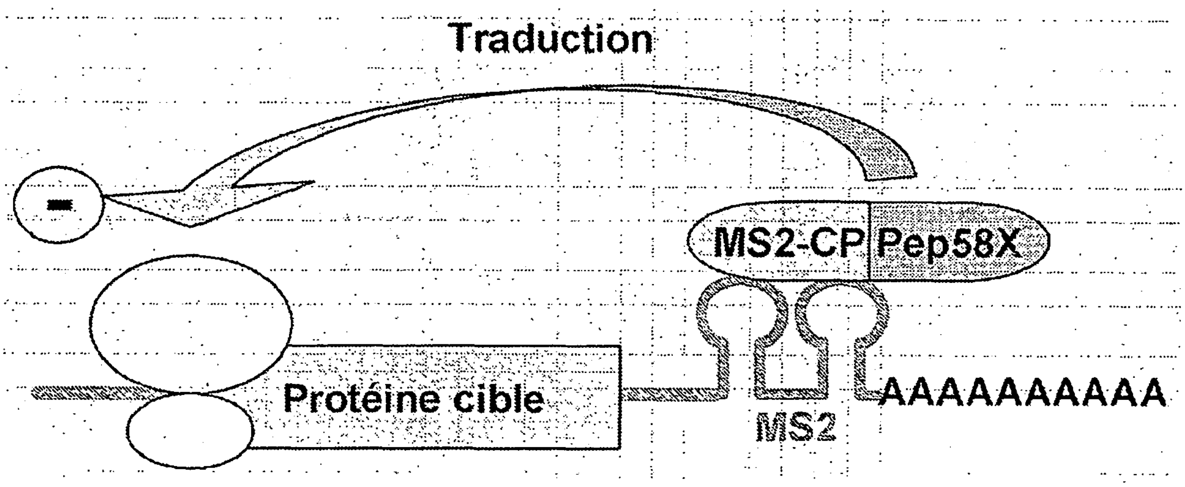


FIGURE 1

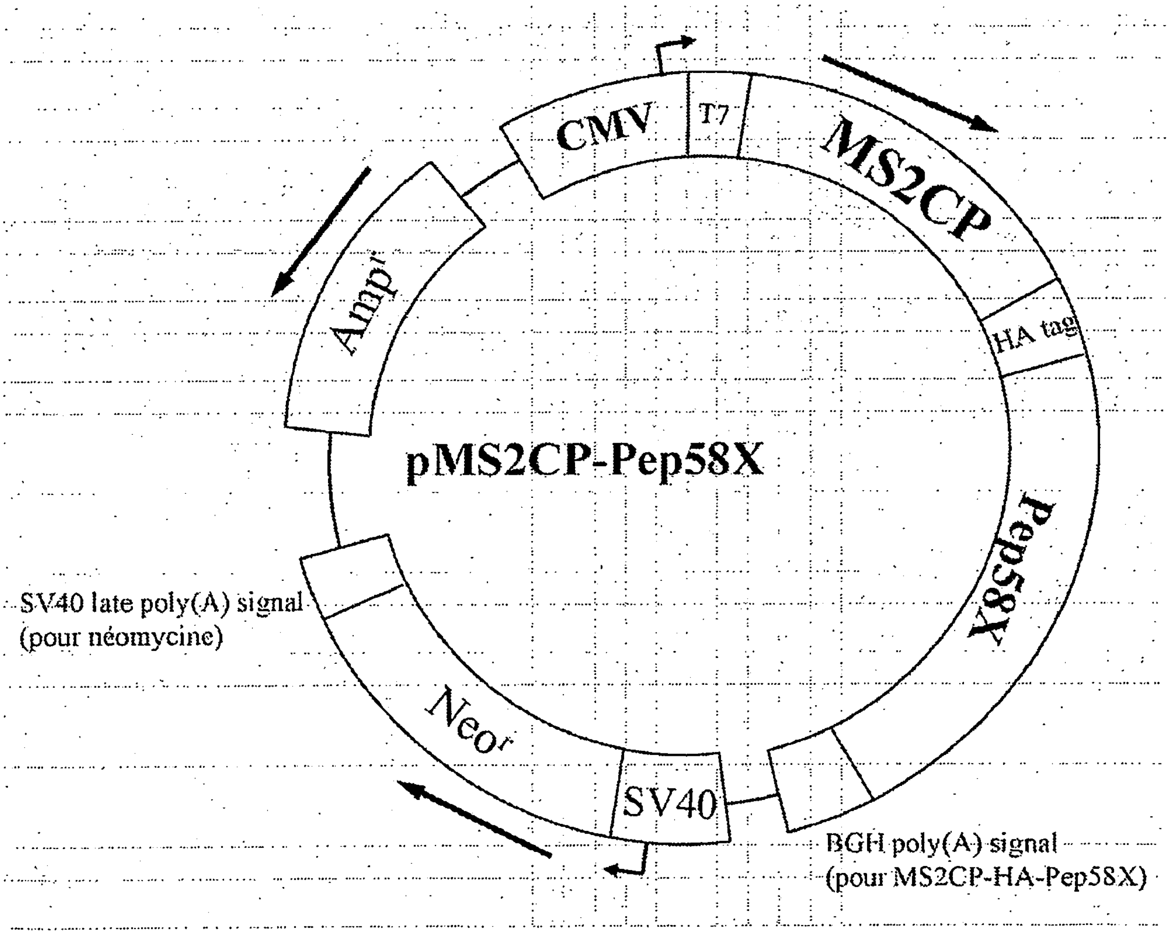
CMV
T7
MS2CP
HA tag
Pep58X
Amp^r
pMS2CP-Pep58X
SV40 late poly(A) signal
(pour néomycine)
Neo^r
SV40
BGH poly(A) signal
(pour MS2CP-HA-Pep58X)

FIGURE 2

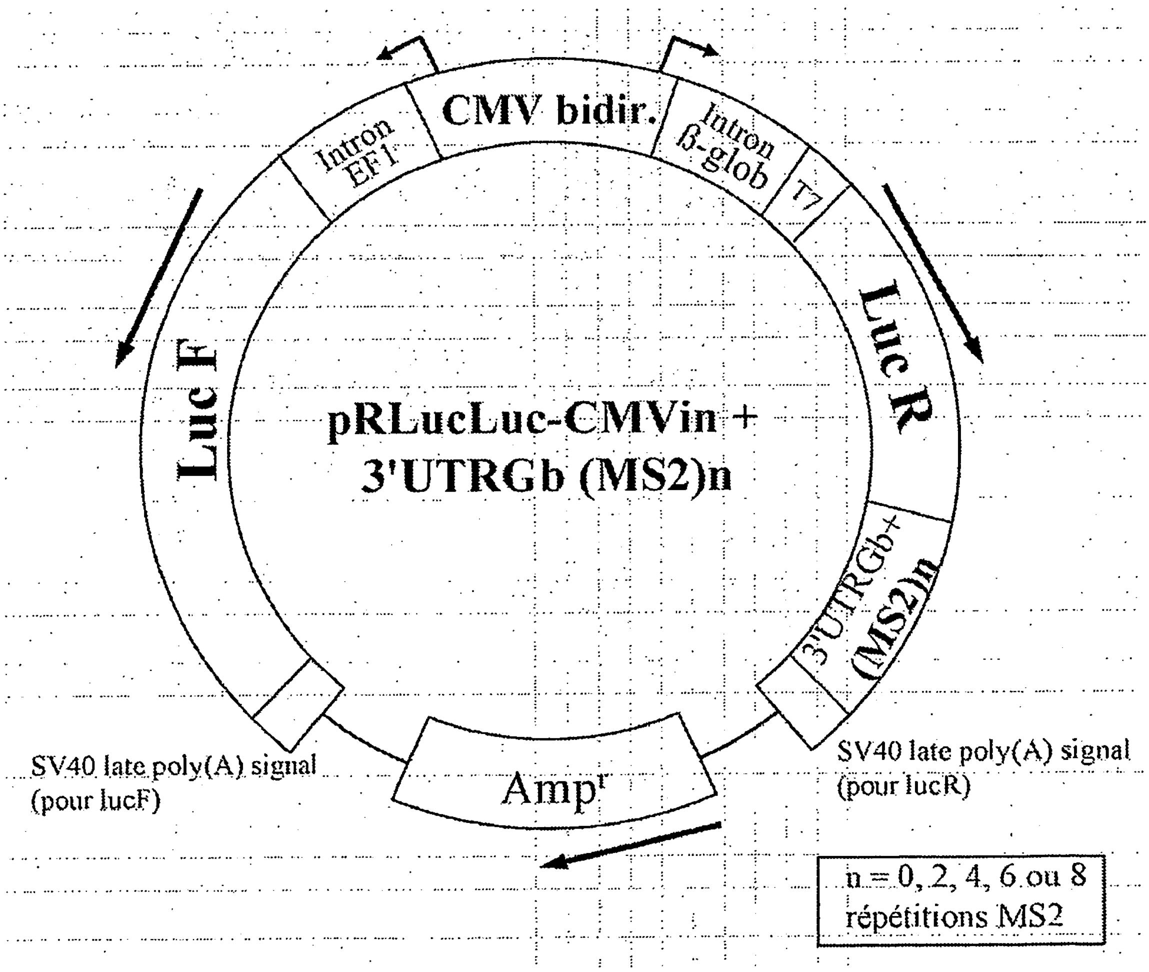
CMV bidir.
Intron EF1
Intron ß-glob
T7
Luc R
Luc F
pRLucLuc-CMVin + 3'UTRGb (MS2)n
3'UTRGb+ (MS2)n
SV40 late poly(A) signal (pour lucF)
SV40 late poly(A) signal (pour lucR)
Amp^r
n = 0, 2, 4, 6 ou 8 répétitions MS2

FIGURE 3

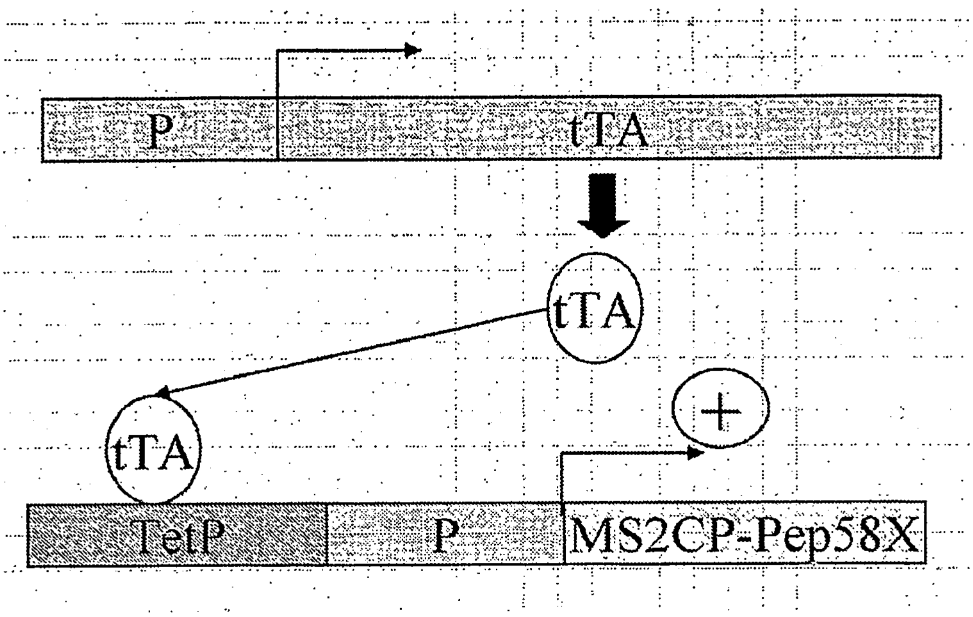


FIGURE 4A

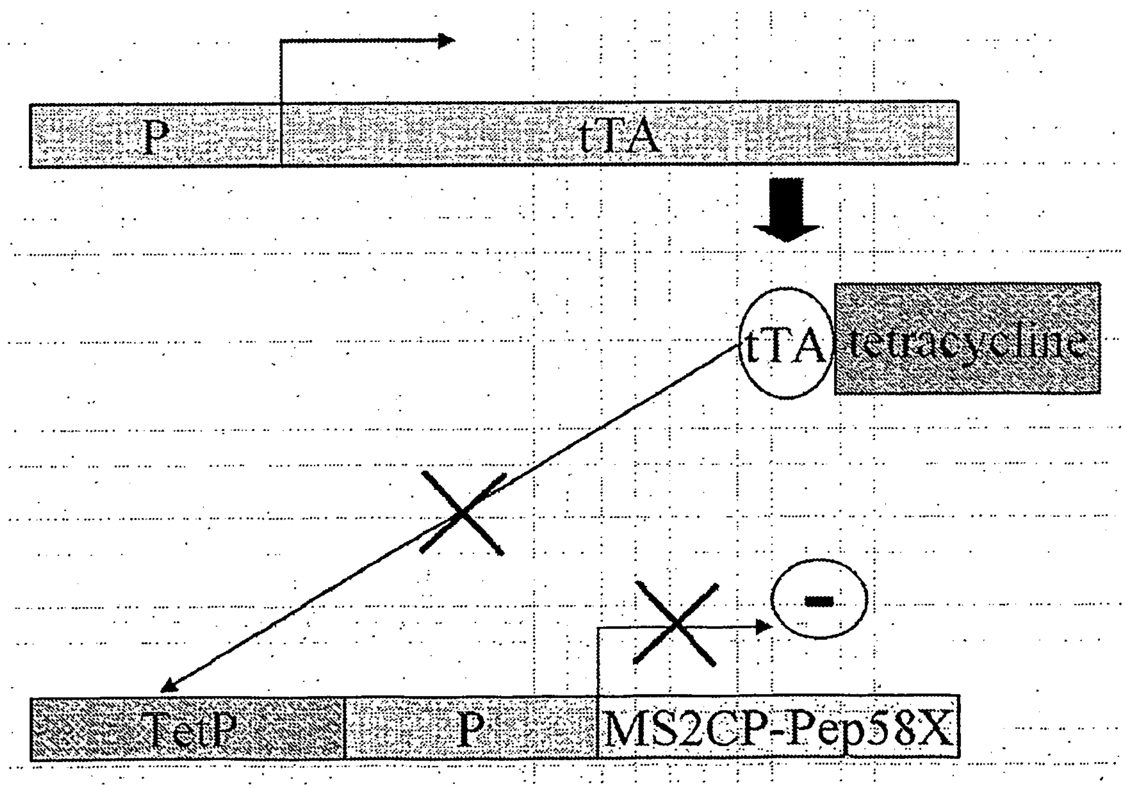


FIGURE 4B

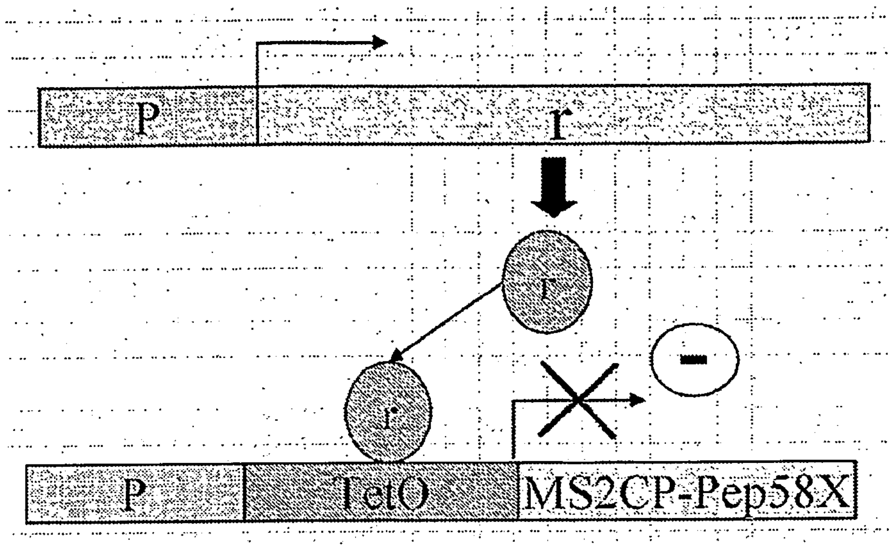


FIGURE 5A

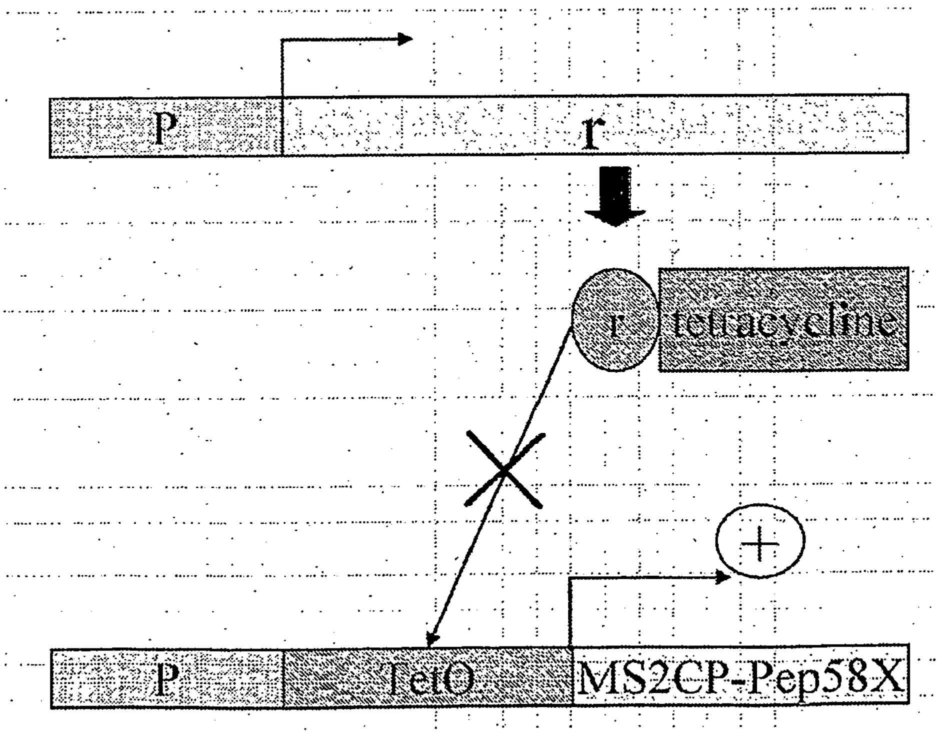


FIGURE 5B

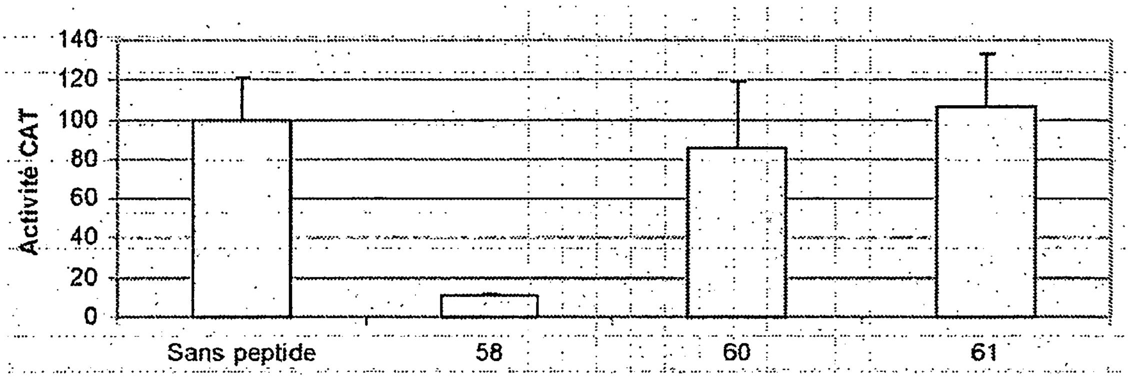


FIGURE 6A

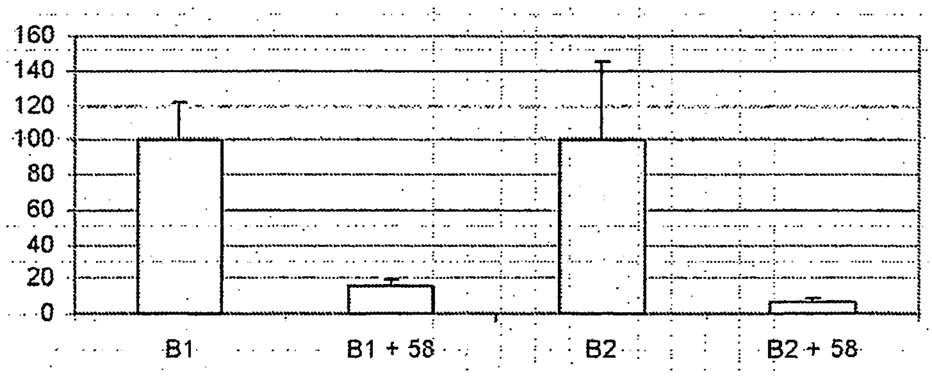


FIGURE 6B

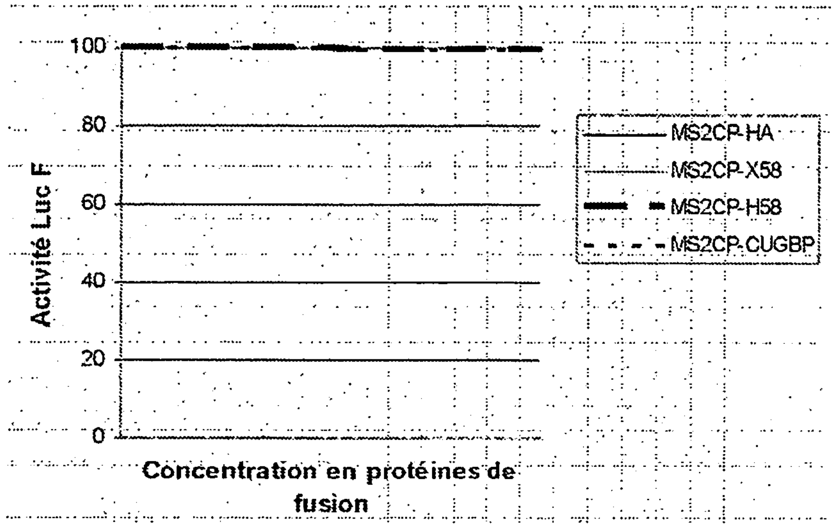


FIGURE 7A

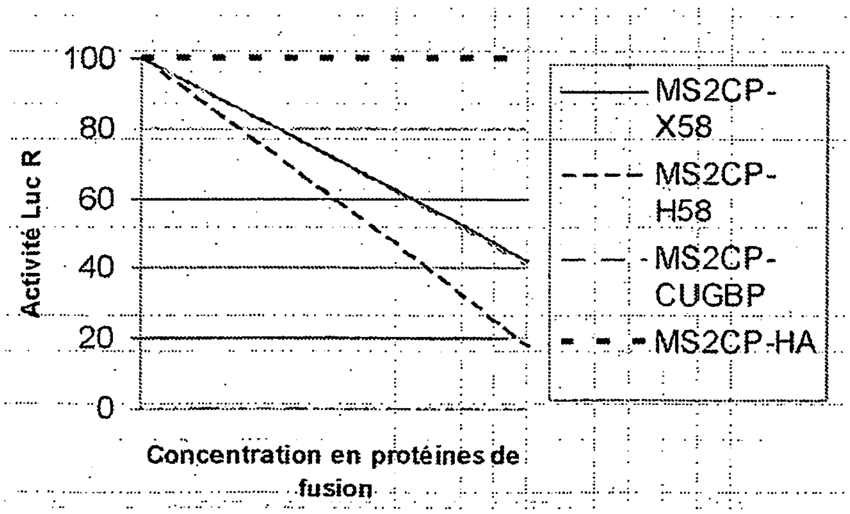


FIGURE 7B

LISTE DE SEQUENCES

<110> Centre National de la Recherche Scientifique

<120> Peptide inhibiteur de la traduction des protéines et utilisation pour le contrôle de la traduction des protéines

<130> P290-FR

<140>
<141>

<160> 16

<170> PatentIn Ver. 2.1

<210> 1
<211> 28
<212> PRT
<213> Xenopus laevis

<400> 1

```
Val Lys Phe Ala Asp Thr Gln Lys Asp Lys Glu Gln Lys Arg Met Thr
 1               5                  10                  15

Gln Gln Leu Gln Gln Gln Met Gln Gln Leu Asn Ala
            20                  25
```

<210> 2
<211> 28
<212> PRT
<213> Homo sapiens

<400> 2

```
Val Lys Phe Ala Asp Thr Gln Lys Asp Lys Glu Gln Lys Arg Met Ala
 1               5                  10                  15

Gln Gln Leu Gln Gln Gln Met Gln Gln Ile Ser Ala
            20                  25
```

<210> 3
<211> 84
<212> PRT
<213> Xenopus laevis

<400> 3

```
Phe Thr Thr Arg Ser Met Ala Gln Met Ala Ile Lys Ser Met His Gln
 1               5                  10                  15

Ala Gln Thr Met Glu Gly Cys Ser Ser Pro Ile Val Val Lys Phe Ala
            20                  25                  30

Asp Thr Gln Lys Asp Lys Glu Gln Lys Arg Met Thr Gln Gln Leu Gln
        35                  40                  45

Gln Gln Met Gln Gln Leu Asn Ala Ala Ser Met Trp Gly Asn Leu Thr
    50                  55                  60

Gly Leu Asn Ser Leu Ala Pro Gln Tyr Leu Ala Leu Leu Gln Gln Thr
 65                  70                  75                  80

Ala Ser Ser Gly
```

<210> 4
<211> 88
<212> PRT
<213> Homo sapiens

<400> 4

```
Phe Thr Thr Arg Ala Met Ala Gln Thr Ala Ile Lys Ala Met His Gln
 1               5                  10                  15

Ala Gln Thr Met Glu Gly Cys Ser Ser Pro Met Val Val Lys Phe Ala
            20                  25                  30

Asp Thr Gln Lys Asp Lys Glu Gln Lys Arg Met Ala Gln Gln Leu Gln
        35                  40                  45

Gln Gln Met Gln Gln Ile Ser Ala Ala Ser Val Trp Gly Asn Leu Ala
    50                  55                  60

Gly Leu Asn Thr Leu Gly Pro Gln Tyr Leu Ala Leu Tyr Leu Gln Leu
 65                  70                  75                  80

Leu Gln Gln Thr Ala Ser Ser Gly
                85
```

<210> 5
<211> 189
<212> PRT
<213> Séquence artificielle

<220>
<223> Description de la séquence artificielle:fusion

<400> 5

```
Met Ala Tyr Pro Tyr Asp Val Pro Asp Tyr Ala Ser Val Lys Phe Ala
1               5                   10                  15

Asp Thr Gln Lys Asp Lys Glu Gln Lys Arg Met Thr Gln Gln Leu Gln
            20                  25                  30

Gln Gln Met Gln Gln Leu Asn Ala Ala Ala Ala Met Ala Ser Asn Phe
        35                  40                  45

Thr Gln Phe Val Leu Val Asp Asn Gly Gly Thr Gly Asp Val Thr Val
    50                  55                  60

Ala Pro Ser Asn Phe Ala Asn Gly Val Ala Glu Trp Ile Ser Ser Asn
65                  70                  75                  80

Ser Arg Ser Gln Ala Tyr Lys Val Thr Cys Ser Val Arg Gln Ser Ser


                85                  90                  95

Ala Gln Asn Arg Lys Tyr Thr Ile Lys Val Glu Val Pro Lys Val Ala
            100                 105                 110

Thr Gln Thr Val Gly Gly Glu Glu Leu Pro Val Ala Gly Trp Arg Ser
        115                 120                 125

Tyr Leu Asn Met Glu Leu Thr Ile Pro Ile Phe Ala Thr Asn Ser Asp
    130                 135                 140

Cys Glu Leu Ile Val Lys Ala Met Gln Gly Leu Leu Lys Asp Gly Asn
145                 150                 155                 160

Pro Ile Pro Ser Ala Ile Ala Ala Asn Ser Gly Ile Tyr Gly Gly Gly
                165                 170                 175

Gly Gly Ser Gly Pro Tyr Ser Ile Val Ser Pro Lys Cys
            180                 185
```

<210> 6
<211> 154
<212> PRT
<213> Séquence artificielle

<220>
<223> Description de la séquence artificielle:fusion

<400> 6

```
Met Ala Ser Asn Phe Thr Gln Phe Val Leu Val Asp Asn Gly Gly Thr
 1               5                  10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Glu Glu Leu Pro Val
65                  70                  75                  80

Ala Gly Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr Gly Gly Gly Gly Gly Ser Lys Leu Gly Ser Met Ala Tyr Pro
    130                 135                 140

Tyr Asp Val Pro Asp Tyr Ala Arg Ala Ala
145                 150
```

<210> 7
<211> 570
<212> ADN
<213> Séquence artificielle

<220>
<223> Description de la séquence artificielle:fusion

<400> 7

```
atggcttacc catacgatgt tccagattac gctagcgtaa agttcgcaga cactcagaaa 60
gacaaagaac agaagcgcat gacgcagcaa cttcagcagc aaatgcagca gctcaatgca 120
gcggccgcca tggcttctaa ctttactcag ttcgttctcg tcgacaatgg cggaactggc 180
gacgtgactg tcgccccaag caacttcgct aacggggtcg ctgaatggat cagctctaac 240
tcgcgatcac aggcttacaa agtaacctgt agcgttcgtc agagctctgc gcagaatcgc 300
aaatacacca tcaaagtcga ggtgcctaaa gtggcaaccc agactgttgg tggtgaagag 360
cttcctgtag ccggatggag atcttactta aatatggaac taaccattcc aattttcgcc 420
acgaattccg actgcgagct tattgttaag gcaatgcaag gtctcctaaa agatggaaac 480
ccgattccct cggccatcgc ggccaactcc ggcatctacg gaggtggagg tggatctggg 540
ccctattcta tagtgtcacc taaatgctag                                  570
```

<210> 8
<211> 570
<212> ADN
<213> Séquence artificielle

<220>
<223> Description de la séquence artificielle:fusion

<400> 8

```
atggcttacc catacgatgt tccagattac gctagcgtaa aatttgctga tacacagaag 60
gacaaagaac agaagagaat ggcccagcag ctccagcagc agatgcagca aatcagcgca 120
gcggccgcca tggcttctaa ctttactcag ttcgttctcg tcgacaatgg cggaactggc 180
gacgtgactg tcgccccaag caacttcgct aacggggtcg ctgaatggat cagctctaac 240
tcgcgatcac aggcttacaa agtaacctgt agcgttcgtc agagctctgc gcagaatcgc 300
aaatacacca tcaaagtcga ggtgcctaaa gtggcaaccc agactgttgg tggtgaagag 360
cttcctgtag ccggatggag atcttactta aatatggaac taaccattcc aattttcgcc 420
acgaattccg actgcgagct tattgttaag gcaatgcaag gtctcctaaa agatggaaac 480
ccgattccct cggccatcgc ggccaactcc ggcatctacg gaggtggagg tggatctggg 540
ccctattcta tagtgtcacc taaatgctag                                  570
```

<210> 9
<211> 489
<212> PRT
<213> Xenopus laevis

<400> 9

```
Met Asn Gly Thr Met Asp His Pro Asp His Pro Asp Pro Asp Ser Ile
1               5                   10                  15

Lys Met Phe Val Gly Gln Val Pro Arg Ser Trp Ser Glu Lys Glu Leu
            20                  25                  30

Arg Glu Leu Phe Glu Gln Tyr Gly Ala Val Tyr Glu Ile Asn Val Leu
        35                  40                  45

Arg Asp Arg Ser Gln Asn Pro Pro Gln Ser Lys Gly Cys Cys Phe Ile
    50                  55                  60
```

```
Thr Phe Tyr Thr Arg Lys Ala Ala Leu Glu Ala Gln Asn Ala Leu His
 65                  70                  75                  80

Asn Met Lys Val Leu Pro Gly Met His His Pro Ile Gln Met Lys Pro
                 85                  90                  95

Ala Asp Ser Glu Lys Asn Asn Ala Val Glu Asp Arg Lys Leu Phe Ile
            100                 105                 110

Gly Met Val Ser Lys Asn Cys Asn Glu Asn Asp Ile Arg Ala Met Phe
        115                 120                 125

Ser Pro Phe Gly Gln Ile Glu Glu Cys Arg Ile Leu Arg Gly Pro Asp
    130                 135                 140

Gly Met Ser Arg Gly Cys Ala Phe Val Thr Phe Thr Thr Arg Ser Met
145                 150                 155                 160

Ala Gln Met Ala Ile Lys Ser Met His Gln Ala Gln Thr Met Glu Gly
                165                 170                 175

Cys Ser Ser Pro Ile Val Val Lys Phe Ala Asp Thr Gln Lys Asp Lys
            180                 185                 190

Glu Gln Lys Arg Met Thr Gln Gln Leu Gln Gln Gln Met Gln Gln Leu
        195                 200                 205

Asn Ala Ala Ser Met Trp Gly Asn Leu Thr Gly Leu Asn Ser Leu Ala
    210                 215                 220

Pro Gln Tyr Leu Ala Leu Leu Gln Gln Thr Ala Ser Ser Gly Asn Leu
225                 230                 235                 240

Asn Ser Leu Ser Gly Leu His Pro Met Gly Ala Glu Tyr Gly Thr Gly
                245                 250                 255

Met Thr Ser Gly Leu Asn Ala Ile Gln Leu Gln Asn Leu Ala Ala Leu
            260                 265                 270

Ala Ala Ala Ala Ser Ala Ala Gln Asn Thr Pro Ser Ala Gly Ala Ala
        275                 280                 285

Leu Thr Ser Ser Ser Ser Pro Leu Ser Ile Leu Thr Ser Ser Gly Ser
    290                 295                 300

Ser Pro Ser Ser Asn Asn Ser Ser Ile Asn Thr Met Ala Ser Leu Gly
305                 310                 315                 320

Ala Leu Gln Thr Leu Ala Gly Ala Thr Ala Gly Leu Asn Val Asn Ser
                325                 330                 335

Leu Ala Gly Met Ala Ala Phe Asn Gly Gly Leu Gly Ser Ser Leu Ser
            340                 345                 350

Asn Gly Thr Gly Ser Thr Met Glu Ala Leu Ser Gln Ala Tyr Ser Gly
        355                 360                 365

Ile Gln Gln Tyr Ala Ala Ala Ala Leu Pro Ser Leu Tyr Asn Gln Ser
    370                 375                 380
```

```
Leu Leu Ser Gln Gln Gly Leu Gly Ala Ala Gly Ser Gln Lys Glu Gly
385                 390                 395                 400

Pro Glu Gly Ala Asn Leu Phe Ile Tyr His Leu Pro Gln Glu Phe Gly
                405                 410                 415

Asp Gln Asp Leu Leu Gln Met Phe Met Pro Phe Gly Asn Val Val Ser
            420                 425                 430

Ser Lys Val Phe Ile Asp Lys Gln Thr Asn Leu Ser Lys Cys Phe Gly
        435                 440                 445

Phe Val Ser Tyr Asp Asn Pro Val Ser Ala Gln Ala Ala Ile Gln Ser
    450                 455                 460

Met Asn Gly Phe Gln Ile Gly Met Lys Arg Leu Lys Val Gln Leu Lys
465                 470                 475                 480

Arg Ser Lys Asn Asp Ser Lys Pro Tyr
                485
```

<210> 10
<211> 1470
<212> ADN
<213> Xenopus laevis

<400> 10

```
atgaatggca caatggacca cccagaccat ccggatccgg actccatcaa gatgtttgtg 60
ggtcaggttc ctcgaagctg gtcagagaaa gagctaagag aactcttcga gcagtacgga 120
gccgtctatg aaattaatgt tctccgagac agaagccaga atcctcctca gagcaaagga 180
tgctgtttta ttactttcta cacaagaaaa gctgcgttag aagcacagaa tgctttgcac 240
aacatgaaag ttctccctgg gatgcatcat ccaatacaga tgaagccagc cgacagtgaa 300
aagaataatg ctgtggaaga ccgaaagcta tttatcggaa tggtttccaa gaattgtaat 360
gagaatgata tccgggccat gttctctccg tttggacaga tagaggaatg tcgtatcctg 420
cgaggccctg atggaatgag cagaggatgt gcattcgtta cgtttacaac tagatccatg 480
gcacagatgg caatcaaatc catgcaccaa gcacaaacca tggagggctg ttcctcacca 540
atagtggtaa agttcgcaga cactcagaaa gacaaagaac agaagcgcat gacgcagcaa 600
cttcagcagc aaatgcagca gctcaatgca gcctcaatgt ggggtaacct gactggactg 660
aacagcttgg caccccagta tttagcactc ctccagcaga ccgcctcctc tgggaacctc 720
aactccctaa gtggtctcca ccctatggga gctgagtacg gcactggaat gacatcaggg 780
cttaatgcca tacagttaca gaatttggca gctttagcgg ctgctgctag tgctgcgcag 840
aacaccccaa gtgcaggagc agcgctcact tcttccagca gccccctcag catcctaacc 900
agttccggtt cctcccccag ttcaaataac tcatccatca acaccatggc atccctagga 960
gctctacaga cattggctgg ggccacagct ggtctcaatg tcaattcgct tgcaggtatg 1020
gctgcgttta atggaggcct aggcagcagt ctctccaatg gcactggcag tacgatggaa 1080
gcccttagtc aagcttactc tgggattcag cagtatgctg ccgctgcact tccttcactc 1140
tataaccaga gccttttgtc acaacagggt ttgggggctg cggggagtca gaaagaaggc 1200
ccagaaggag ccaacctttt tatataccac ctaccccagg agtttgggga ccaggatctc 1260
ctgcagatgt tcatgccatt tggaaatgtt gtgtcctcca aagttttcat cgacaaacaa 1320
acgaacctca gcaaatgttt tggcttcgta agttacgaca atcccgtttc tgctcaggct 1380
gctatccagt ccatgaacgg ctttcagatc ggaatgaaac gcctgaaagt ccaactcaaa 1440
cgctccaaga atgacagcaa accctactga                                 1470
```

<210> 11
<211> 9
<212> PRT
<213> Séquence artificielle

<220>

<223> Description de la séquence artificielle:peptide HA

<400> 11

```
Tyr Pro Tyr Asp Val Pro Asp Tyr Ala
 1               5
```

<210> 12
<211> 33
<212> ADN
<213> Séquence artificielle

<220>
<223> Description de la séquence artificielle:amorce

<400> 12
atgctagcgt aaagttcgca gacactcaga aag 33

<210> 13
<211> 32
<212> ADN
<213> Séquence artificielle

<220>
<223> Description de la séquence artificielle:amorce

<400> 13
atgcggccgc tgcattgagc tgctgcattt gc 32

<210> 14
<211> 32
<212> ADN
<213> Séquence artificielle

<220>
<223> Description de la séquence artificielle:amorce

<400> 14
atgctagcgt aaaatttgct gatacacaga ag 32

<210> 15
<211> 32
<212> ADN
<213> Séquence artificielle

<220>
<223> Description de la séquence artificielle:amorce

<400> 15
atgcggccgc tgcgctgatt tgctgcatct gc 32

<210> 16
<211> 27
<212> ADN
<213> Séquence artificielle

<220>

<223> Description de la séquence artificielle:peptide HA

<400> 16
tacccatacg atgttccaga ttacgct 27